(19) Europäisches Patentamt
European Patent Office
Office européen des brevets

(11) EP 3 620 053 A1

(12) **EUROPEAN PATENT APPLICATION**

(43) Date of publication:
**11.03.2020 Bulletin 2020/11**

(21) Application number: **18212562.5**

(22) Date of filing: **14.12.2018**

(51) Int Cl.:
***A01N 43/653*** *(2006.01)* ***C07D 401/06*** *(2006.01)*
***C07D 249/08*** *(2006.01)* ***C07D 211/74*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 10**
**40789 Monheim am Rhein (DE)**

(54) **FUNGICIDAL ACTIVE COMPOUND COMBINATIONS**

(57) The present invention relates to active compound combinations comprising at least one triazole derivative of formula (I)

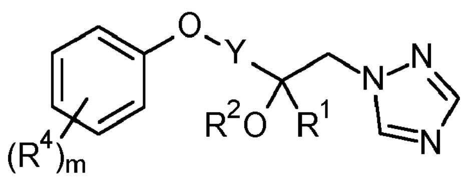

(I)

as defined in claim 1 and the further fungicide [(1S)-2,2-bis(4-fluorophenyl)-1-methyl-ethyl](2S)-2-[(3-acetoxy-4-methoxy-pyridine-2-carbonyl)amino]propanoate (II), to compositions comprising such compound combination, and to the use thereof as biologically active agents, especially for control of harmful microorganisms in crop protection and in the protection of materials.

EP 3 620 053 A1

## Description

The present invention relates to active compound combinations, in particular within a fungicide composition, which comprises (A) a triazole derivative of formula (I) and a further fungicidally active compound (B). Moreover, the invention relates to compositions comprising such compound combination and to the use thereof as biologically active combinations, especially for control of harmful microorganisms, in particular fungi, in crop protection and in the protection of materials and as plant growth regulators.

It is already known that particular phenoxy-phenyl-substituted triazole derivatives can be used in crop protection as fungicides (e.g. EP-A 0 275 955; J. Agric. Food Chem. 2009, 57, 4854-4860; CN-A 101225074, DE-A 40 03 180; EP-A 0 113 640; EP-A 0 470 466; US 4,949,720; EP-A 0 126 430, DE-A 38 01 233; WO-A 2013/007767; WO-A 2013/010862; WO-A 2013/010885; WO-A 2013/010894; WO-A 2013/024075; WO-A 2013/024076; WO-A 2013/024077; WO-A 2013/024080; WO-A 2013/024081; WO-A 2013/024082; WO-A 2013/024083 and WO-A 2014/082872). Also particular phenoxy-phenyl-substituted triazolinethione derivatives (e.g. WO-A 2010/146114) and particular phenoxy-hetaryl-substituted triazolinethione derivatives (e.g. WO-A 2010/146116) are known to be useful in crop protection as fungicides. Furthermore, particular phenoxy-hetaryl-substituted triazole derivatives are known from WO-A 2017/029179.

Since the environmental and economic requirements imposed on modern-day crop protection agents and compositions are continually increasing, with regard, for example, to the spectrum of action, safety profile, selectivity, application rate, formation of residues, and favourable preparation ability, and since, furthermore, there may be problems, for example, with resistances, a constant task is to develop new compositions, in particular fungicidal agents, which in some areas at least help to fulfil the abovementioned requirements. The present invention provides active compound combinations and compositions comprising said combinations which at least in some aspects achieve the stated objective.

Accordingly, the present invention provides active compound combinations comprising

(A) at least one triazole derivative of formula (I)

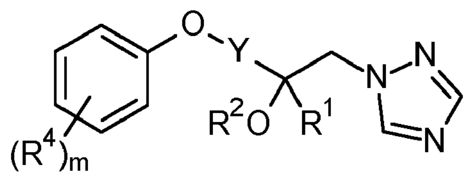

(I),

wherein

$R^1$ represents hydrogen, $C_1$-$C_6$-alkyl, $C_2$-$C_6$-alkenyl, $C_2$-$C_6$-alkynyl, $C_3$-$C_8$-cycloalkyl, $C_3$-$C_8$-cycloalkyl-$C_1$-$C_4$-alkyl or phenyl;

$R^2$ represents hydrogen, $C_1$-$C_6$-alkyl, $C_2$-$C_6$-alkenyl, $C_2$-$C_6$-alkynyl, $C_3$-$C_8$-cycloalkyl, $C_3$-$C_8$-cycloalkyl-$C_1$-$C_4$-alkyl or phenyl,
wherein the aliphatic moieties, excluding cycloalkyl moieties, of $R^1$ and $R^2$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen, CN, nitro, $C_1$-$C_4$-alkyl, $C_1$-$C_4$-alkoxy, $C_1$-$C_4$-haloalkyl, $C_1$-$C_4$-haloalkoxy,
and wherein the cycloalkyl and/or phenyl moieties of $R^1$ and $R^2$ may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-haloalkyl and $C_1$-$C_4$-haloalkoxy;

each $R^4$ represents independently of one another halogen, CN, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylcarbonyl, hydroxy-substituted $C_1$-$C_4$-alkyl, pentafluoro-$\lambda^6$-sulfanyl, $C_3$-$C_6$-cycloalkyl, $C_3$-$C_6$-halocycloalkyl, $C_1$-$C_4$-alkyl-$C_3$-$C_6$-cycloalkyl, $C_2$-$C_6$-alkenyl, $C_2$-$C_6$-haloalkenyl, $C_2$-$C_6$-alkynyl, $C_2$-$C_6$-haloalkynyl, $C_1$-$C_4$-alkylsulfanyl, $C_1$-$C_4$-haloalkylsulfanyl, $C_1$-$C_6$-alkylsulfonyl, $C_6$-$C_{10}$-arylsulfonyl, $C_1$-$C_6$-alkyl-$SO_2NH$-, $C_6$-$C_{10}$-aryl-$SO_2NH$-, formyl, 5-, 6- or 7-membered saturated heterocycloalkyl containing up to 4 heteroatoms selected from N, O and S, or -C($R^{4a}$)=N-O$R^{4b}$, wherein $R^{4a}$ and $R^{4b}$ represent independently from each other hydrogen, $C_1$-$C_6$-alkyl or phenyl;

m is an integer and is 0, 1, 2, 3, 4 or 5;

Y represents a 6-membered aromatic cycle selected from

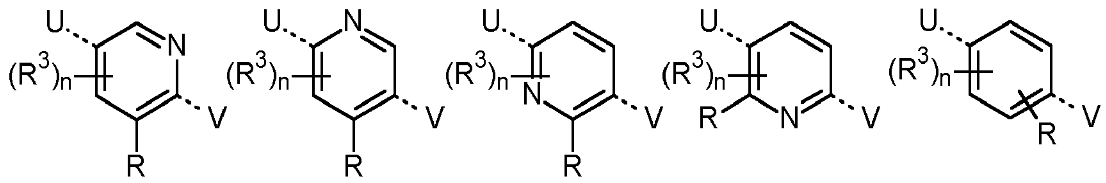

wherein Y is connected to the O of formula (I) via the bonds identified with "U" and Y is connected to the $CR^1(OR^2)$ moiety of formula (I) via the bonds identified with "V" and wherein

R represents hydrogen, $C_1$-$C_2$-haloalkyl, $C_1$-$C_2$-haloalkoxy, $C_1$-$C_2$-alkylcarbonyl or halogen; preferably hydrogen, $C_1$-$C_2$-haloalkyl or halogen;

each $R^3$ represents independently of one another halogen, CN, nitro, $C_1$-$C_4$-alkyl, $C_1$-$C_4$-haloalkyl, $C_1$-$C_4$-alkoxy or $C_1$-$C_4$-haloalkoxy;

n is an integer and is 0, 1 or 2;

or a salt or N-oxide thereof, and

(B) florylpicoxamid or a salt or N-oxide thereof.

The active compound combinations according to the invention comprise (A) at least one triazole derivative of formula (I) or a salt or N-oxide thereof. The salts or N-oxides of the triazole derivatives of formula (I) also have fungicidal properties.

Formula (I) provides a general definition of the triazole derivatives present in compound combinations according to the invention. Preferred radical definitions for the formulae shown above and below are given below. These definitions apply to the end products of the formula (I) and likewise to all intermediates.

$R^1$ preferably represents hydrogen, $C_1$-$C_4$-alkyl, $C_2$-$C_6$-alkenyl, $C_2$-$C_6$-alkynyl, cyclopropyl or phenyl, wherein the aliphatic moieties, excluding the cycloalkyl moieties, of $R^1$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen, CN, nitro, $C_1$-$C_4$-alkyl, $C_1$-$C_4$-alkoxy, $C_1$-$C_4$-haloalkyl, $C_1$-$C_4$-haloalkoxy;
wherein the cycloalkyl and/or phenyl moieties of $R^1$ may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-haloalkyl and $C_1$-$C_4$-haloalkoxy.

$R^1$ more preferably represents hydrogen, methyl, ethyl, propyl, isopropyl, butyl, cyclopropyl, $CF_3$, allyl, $CH_2C{\equiv}C-CH_3$ or $CH_2C{\equiv}CH$,
wherein the aliphatic moieties, excluding the cycloalkyl moieties, of $R^1$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen; CN; nitro; $C_1$-$C_4$-alkyl; $C_1$-$C_4$-alkoxy; $C_1$-$C_4$-haloalkyl; $C_1$-$C_4$-haloalkoxy.

$R^1$ more preferably represents hydrogen or non-substituted methyl, ethyl, propyl, isopropyl, butyl, cyclopropyl, $CF_3$, allyl, $CH_2C{\equiv}C-CH_3$ or $CH_2C{\equiv}CH$.

$R^1$ more preferably represents hydrogen, methyl, ethyl or cyclopropyl.

$R^1$ even more preferably represents methyl or cyclopropyl.

$R^1$ most preferably represents methyl.

$R^2$ preferably represents hydrogen, $C_1$-$C_4$-alkyl, allyl or prop-2-inyl, wherein the aliphatic moieties, excluding the cycloalkyl moieties, of $R^2$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen, CN, nitro, $C_1$-$C_4$-alkyl, $C_1$-$C_4$-alkoxy, $C_1$-$C_4$-haloalkyl, $C_1$-$C_4$-haloalkoxy;
wherein the cycloalkyl and/or phenyl moieties of $R^2$ may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-haloalkyl and $C_1$-$C_4$-haloalkoxy.

$R^2$ more preferably represents hydrogen, methyl, ethyl, isopropyl or allyl, wherein the aliphatic moieties, excluding the cycloalkyl moieties, of $R^2$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen, CN, nitro, $C_1$-$C_4$-alkyl, $C_1$-$C_4$-alkoxy, $C_1$-$C_4$-haloalkyl, $C_1$-$C_4$-haloalkoxy.

$R^2$ more preferably represents hydrogen or non-substituted methyl, ethyl, isopropyl or allyl.

$R^2$ more preferably represents hydrogen or methyl.

$R^2$ most preferably represents hydrogen.

Each $R^4$ preferably represents independently from each other halogen, CN, nitro, $C_1$-$C_4$-alkyl, $C_1$-$C_4$-haloalkyl, $C_1$-$C_4$-alkoxy, $C_1$-$C_4$-haloalkoxy or pentafluoro-$\lambda^6$-sulfanyl.

Each $R^4$ more preferably represents independently from each other $CF_3$, $OCF_3$, Br, Cl or pentafluoro-$\lambda^6$-sulfanyl.

$R^4$ more preferably represents $CF_3$, $OCF_3$, Br, Cl or pentafluoro-$\lambda^6$-sulfanyl in the 4-position of the phenyl moiety of formula (I).

$R^4$ even more preferably represents Cl or Br, most preferably Cl or Br in the 4-position of the phenyl moiety of formula (I).

m preferably is 1, 2 or 3.

m more preferably is 1 or 2.

m most preferably is 1.

Y preferably represents

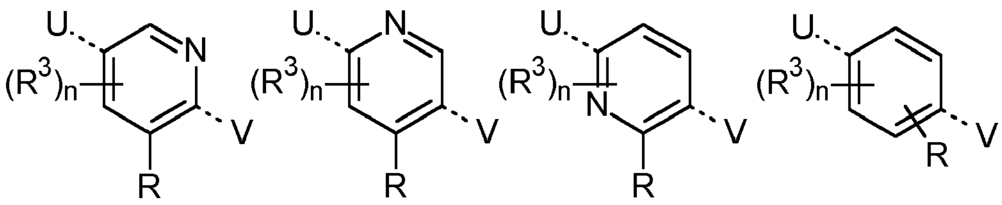

,

wherein
R, $R^3$ and n are defined as mentioned above for formula (I).
R preferably represents hydrogen, $C_1$-haloalkyl, F or Cl.
R more preferably represents $C_1$-haloalkyl, F or Cl.
R more preferably represents $CF_3$ or Cl.
R most preferably represents $CF_3$.
n preferably is 0.

In one preferred embodiment Y represents

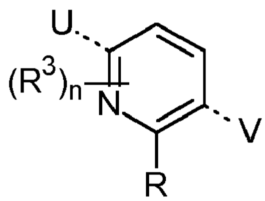

,

wherein

R, $R^3$ and n are defined as mentioned above for formula (I).

R preferably represents hydrogen, $C_1$-haloalkyl, F or Cl.

R more preferably represents $C_1$-haloalkyl, F or Cl.

R more preferably represents $CF_3$ or Cl.

R most preferably represents $CF_3$.

n preferably is 0.

In another preferred embodiment Y represents

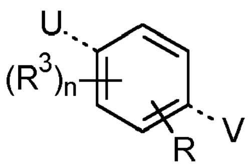

, preferably

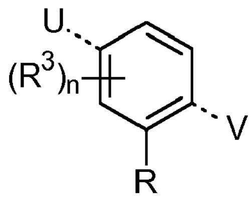

,

wherein

R, $R^3$ and n are defined as mentioned above for formula (I).

R preferably represents hydrogen, $C_1$-haloalkyl, F or Cl.

R more preferably represents $C_1$-haloalkyl, F or Cl.

R more preferably represents $CF_3$ or Cl.

R most preferably represents $CF_3$.

n preferably is 0.

The radical definitions and explanations given above in general terms or stated within preferred ranges can, however, also be combined with one another as desired, i.e. including between the particular ranges and preferred ranges. They apply both to the end products and correspondingly to precursors and intermediates. In addition, individual definitions may not apply.

Preference is given to presence of those compounds of formula (I) in which each of the radicals have the abovementioned preferred definitions.

Particular preference is given to presence of those compounds of formula (I) in which each of the radicals have the abovementioned more and/or most preferred definitions.

Table 1 illustrates examples of compounds according to formula (I) which are particular useful as component (A) of active compound combinations according to the invention.

Table 1: Compounds according to formula (I)

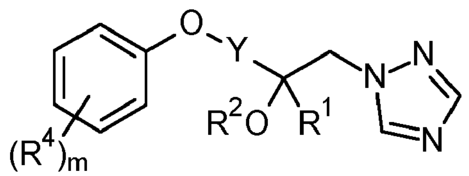

(I)

| Ex N° | Y | n | $R^3$ | R | $R^1$ | $R^2$ | m | $R^4$ |
|---|---|---|---|---|---|---|---|---|
| I.01 | 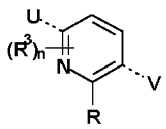 | 0 | - | $CF_3$ | $CH_3$ | H | 1 | 4-Cl |
| I.02 (*) | 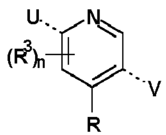 | 0 | - | $CF_3$ | $CH_3$ | H | 1 | 4-Cl |
| I.03 | 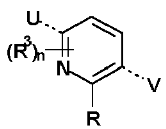 | 0 | - | $CF_3$ | H | H | 1 | 4-Cl |
| I.04 | 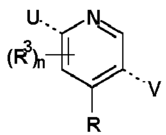 | 0 | - | $CF_3$ | H | H | 1 | 4-Cl |
| I.05 | 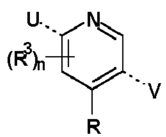 | 0 | - | Cl | $CH_3$ | H | 0 | - |
| I.06 | 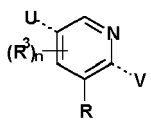 | 0 | - | Cl | $CH_3$ | H | 0 | - |

(continued)

| Ex N° | Y | n | $R^3$ | R | $R^1$ | $R^2$ | m | $R^4$ |
|---|---|---|---|---|---|---|---|---|
| I.07 | 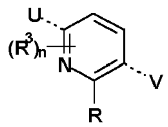 | 0 | - | Cl | $CH_3$ | H | 0 | - |
| I.08 (*) | 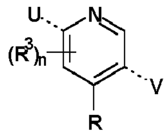 | 0 | - | Cl | $CH_3$ | H | 1 | 4-Cl |
| I.09 (*) | 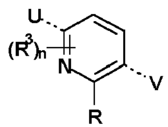 | 0 | - | Cl | $CH_3$ | H | 1 | 4-Cl |
| I.10 | 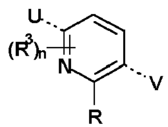 | 0 | - | Cl | H | H | 2 | 3-Cl, 4-Cl |
| I.11 | 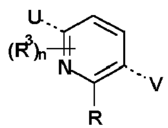 | 0 | - | Cl | H | H | 2 | 3-Cl, 4-$OCF_3$ |
| I.12 | 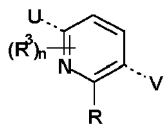 | 0 | - | Cl | H | H | 1 | 4-$OCF_3$ |
| I.13 | 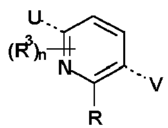 | 0 | - | Cl | H | H | 0 | - |

(continued)

| Ex N° | Y | n | $R^3$ | R | $R^1$ | $R^2$ | m | $R^4$ |
|---|---|---|---|---|---|---|---|---|
| I.14 | 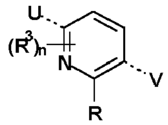 | 0 | - | Cl | H | H | 1 | 4-Cl |
| I.15 | 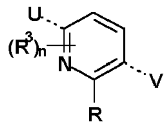 | 0 | - | Cl | H | H | 2 | 2-Me, 4-Cl |
| I.16 | 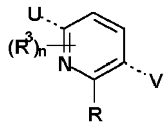 | 0 | - | Cl | H | H | 2 | 3-Cl, 4-Me |
| I.17 | 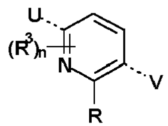 | 0 | - | Cl | H | H | 1 | 4-F |
| I.18 | 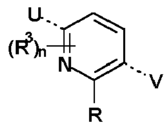 | 0 | - | Cl | H | H | 2 | 2-F, 4-F |
| I.19 | 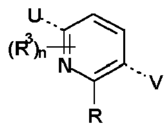 | 0 | - | Cl | H | H | 1 | 2-F |
| I.20 | 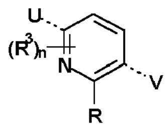 | 0 | - | Cl | H | H | 3 | 3-F, 4-F, 5-F |

(continued)

| Ex N° | Y | n | $R^3$ | R | $R^1$ | $R^2$ | m | $R^4$ |
|---|---|---|---|---|---|---|---|---|
| I.21 | 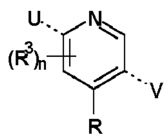 | 0 | - | Cl | H | H | 0 | - |
| I.22 | 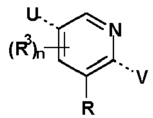 | 0 | - | Cl | H | H | 0 | - |
| I.23 | 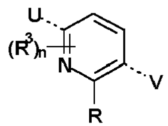 | 0 | - | Cl | H | H | 2 | 2-Cl, 4-Cl |
| I.24 | 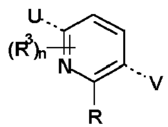 | 0 | - | Cl | H | H | 1 | 4-(1-hydroxyethyl) |
| I.25 | 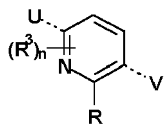 | 0 | - | $CHF_2$ | H | H | 1 | 4-Cl |
| I.26 | 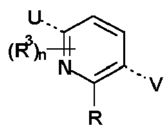 | 0 | - | $OCF_3$ | H | H | 1 | 4-Cl |
| I.27 | 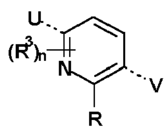 | 0 | - | Cl | $CH_3$ | H | 1 | 4-F |

(continued)

| Ex N° | Y | n | $R^3$ | R | $R^1$ | $R^2$ | m | $R^4$ |
|---|---|---|---|---|---|---|---|---|
| I.28 | 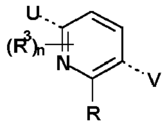 | 0 | - | Cl | $CH_3$ | H | 2 | 2-Me, 4-Cl |
| I.29 | 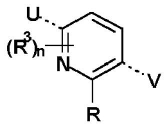 | 0 | - | Cl | $CH_3$ | H | 2 | 3-Cl, 4-Me |
| I.30 | 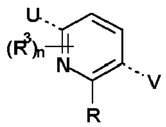 | 0 | - | Cl | H | H | 2 | 2-Cl, 4-OMe |
| I.31 | 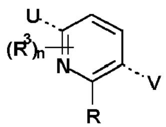 | 0 | - | Cl | $CH_3$ | H | 2 | 2-Cl, 4-OMe |
| I.32 | 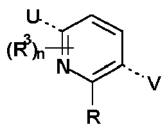 | 0 | - | Cl | $CH_3$ | H | 2 | 3-Cl, 4-Cl |
| I.33 | 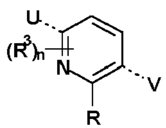 | 0 | - | $OCF_3$ | $CH_3$ | H | 1 | 4-Cl |
| I.34 | 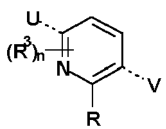 | 0 | - | Cl | H | H | 1 | $4-SF_5$ |

(continued)

| Ex N° | Y | n | $R^3$ | R | $R^1$ | $R^2$ | m | $R^4$ |
|---|---|---|---|---|---|---|---|---|
| I.35 | 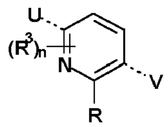 | 0 | - | Cl | $CH_3$ | H | 1 | 4-$SF_5$ |
| I.36 (*) | 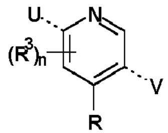 | 0 | - | $CF_3$ | H | H | 1 | 4-$SF_5$ |
| I.37 | 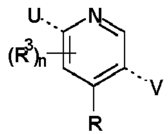 | 0 | - | $CF_3$ | $CH_3$ | H | 1 | 4-$SF_5$ |
| I.38 | 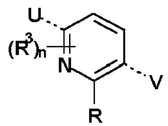 | 0 | - | Cl | H | H | 1 | 4-$CHF_2$ |
| I.39 | 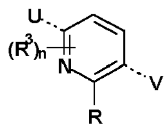 | 0 | - | Cl | $CH_3$ | H | 1 | 4-$CHF_2$ |
| I.40 | 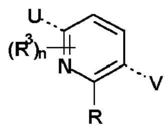 | 0 | - | $CF_3$ | H | H | 1 | 4-Br |
| I.41 | 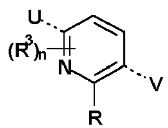 | 0 | - | F | H | H | 1 | 4-Cl |

(continued)

| Ex N° | Y | n | $R^3$ | R | $R^1$ | $R^2$ | m | $R^4$ |
|---|---|---|---|---|---|---|---|---|
| I.42 | 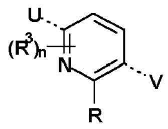 | 0 | - | F | $CH_3$ | H | 1 | 4-Cl |
| I.43 | 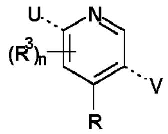 | 0 | - | $CF_3$ | H | H | 1 | 4-$OCF_3$ |
| I.44 | 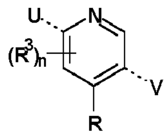 | 0 | - | $CF_3$ | $CH_3$ | H | 1 | 4-$OCF_3$ |
| I.45 (*) | 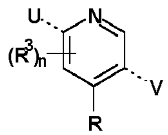 | 0 | - | $CF_3$ | $CH_3$ | H | 1 | 4-Cl |
| I.46 (*) | 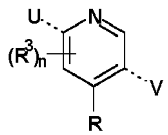 | 0 | - | $CF_3$ | $CH_3$ | H | 1 | 4-Cl |
| I.47 (*) | 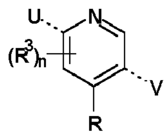 | 0 | - | $CF_3$ | H | H | 1 | 4-$CF_3$ |
| I.48 | 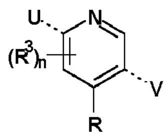 | 0 | - | $CF_3$ | H | H | 1 | 4-Br |

(continued)

| Ex N° | Y | n | $R^3$ | R | $R^1$ | $R^2$ | m | $R^4$ |
|---|---|---|---|---|---|---|---|---|
| I.49 | 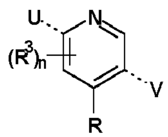 | 0 | - | $CF_3$ | $CH_3$ | H | 1 | 4-Br |
| I.50 | 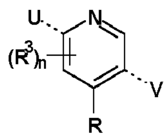 | 0 | - | $CF_3$ | $CH_3$ | H | 1 | 4-$CF_3$ |
| I.51 (*) | 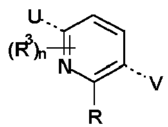 | 0 | - | Cl | $CH_3$ | H | 1 | 4-Cl |
| I.52 (*) | 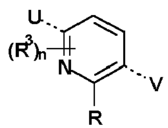 | 0 | - | Cl | $CH_3$ | H | 1 | 4-Cl |
| I.53 (*) | 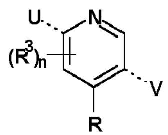 | 0 | - | Cl | $CH_3$ | H | 1 | 4-Cl |
| I.54 (*) | 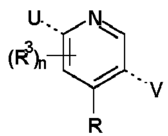 | 0 | - | Cl | $CH_3$ | H | 1 | 4-Cl |
| I.55 | 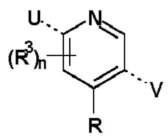 | 0 | - | $CF_3$ | H | H | 1 | 4-$CHF_2$ |

(continued)

| Ex N° | Y | n | $R^3$ | R | $R^1$ | $R^2$ | m | $R^4$ |
|---|---|---|---|---|---|---|---|---|
| I.56 | 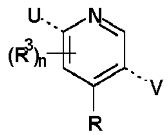 | 0 | - | $CF_3$ | $CH_3$ | H | 1 | 4-$CHF_2$ |
| I.57 | 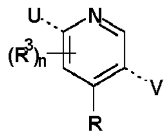 | 0 | - | $CF_3$ | H | H | 2 | 2-F, 4-Cl |
| I.58 | 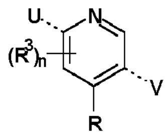 | 0 | - | $CF_3$ | $CH_3$ | H | 2 | 2-F, 4-Cl |
| I.59 (*) | 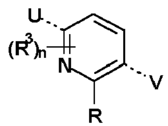 | 0 | - | $CF_3$ | $CH_3$ | H | 1 | 4-Br |
| I.60 | 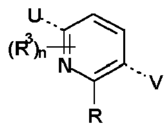 | 0 | - | $CF_3$ | H | H | 1 | 4-$SF_5$ |
| I.61 | 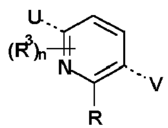 | 0 | - | $CF_3$ | H | H | 1 | 4-$CF_3$ |
| I.62 | 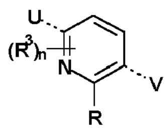 | 0 | - | $CF_3$ | $CH_3$ | H | 1 | 4-$SF_5$ |

(continued)

| Ex N° | Y | n | R$^3$ | R | R$^1$ | R$^2$ | m | R$^4$ |
|---|---|---|---|---|---|---|---|---|
| I.63 | 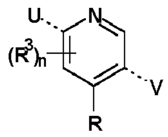 | 0 | - | $CHF_2$ | H | H | 1 | 4-Br |
| I.64 | 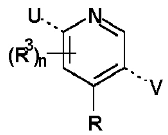 | 0 | - | $CHF_2$ | $CH_3$ | H | 1 | 4-Cl |
| I.65 | 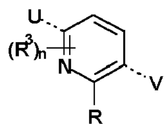 | 0 | - | $CF_3$ | $CH_3$ | H | 1 | 4-$CF_3$ |
| I.66 | 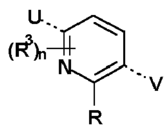 | 0 | - | $CF_3$ | $CH_3$ | H | 2 | 2-F, 4-Cl |
| I.67 | 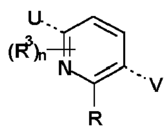 | 0 | - | $CF_3$ | $CH_3$ | H | 1 | 4-Cl |
| I.68 | 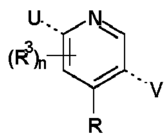 | 0 | - | Cl | H | H | 1 | 4-Cl |
| I.69 | 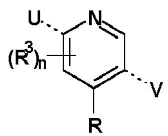 | 0 | - | $CF_3$ | vinyl | H | 1 | 4-$CF_3$ |

(continued)

| Ex N° | Y | n | $R^3$ | R | $R^1$ | $R^2$ | m | $R^4$ |
|---|---|---|---|---|---|---|---|---|
| I.70 | 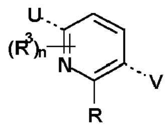 | 0 | - | $CF_3$ | H | H | 2 | 2-F, 4-Cl |
| I.71 | 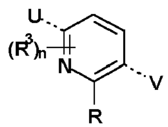 | 0 | - | $CF_3$ | cyclopropyl | H | 1 | 4-Cl |
| I.72 (*) | 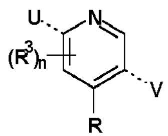 | 0 | - | $CF_3$ | H | H | 1 | 4-$CF_3$ |
| I.73 | 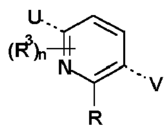 | 0 | - | $CHF_2$ | H | H | 1 | 4-Br |
| I.74 | 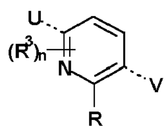 | 0 | - | $CF_3$ | H | H | 1 | 4-$CHF_2$ |
| I.75 | 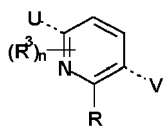 | 0 | - | $CF_3$ | $CH_3$ | H | 1 | 4-isopropyl |
| I.76 | 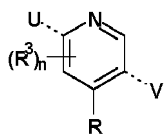 | 0 | - | $CF_3$ | Et | H | 1 | 4-$CF_3$ |

(continued)

| Ex N° | Y | n | R³ | R | R¹ | R² | m | R⁴ |
|---|---|---|---|---|---|---|---|---|
| I.77 | 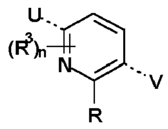 | 0 | - | 4-fluoropheno xy | $CH_3$ | H | 1 | 4-F |
| I.78 | 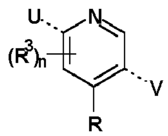 | 0 | - | Cl | $CH_3$ | H | 1 | 4-$CF_3$ |
| I.79 | 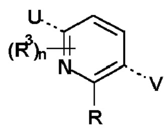 | 0 | - | $CF_3$ | H | H | 1 | 4-$OCF_3$ |
| I.80 | 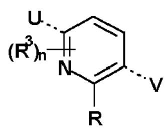 | 0 | - | $CF_3$ | cyclopropyl | H | 1 | 4-Cl |
| I.81 (*) | 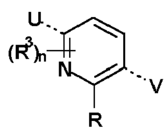 | 0 | - | $CF_3$ | cyclopropyl | H | 1 | 4-Br |
| I.82 | 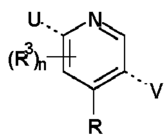 | 0 | - | $CHF_2$ | $CH_3$ | H | 1 | 4-Br |
| I.83 (*) | 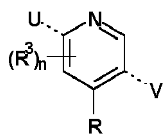 | 0 | - | $CF_3$ | H | H | 1 | 4-$SF_5$ |

(continued)

| Ex N° | Y | n | $R^3$ | R | $R^1$ | $R^2$ | m | $R^4$ |
|---|---|---|---|---|---|---|---|---|
| I.84 (*) | 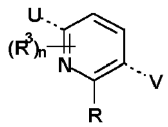 | 0 | - | $CF_3$ | cyclopropyl | H | 1 | 4-Br |
| I.85 | 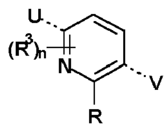 | 0 | - | $CF_3$ | $CF_3$ | H | 1 | 4-Br |
| I.86 (*) | 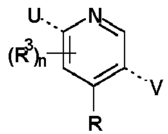 | 0 | - | $CF_3$ | H | H | 1 | 4-$SF_5$ |
| I.87 | 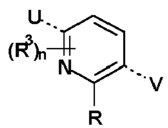 | 0 | - | $CF_3$ | cyclopropyl | H | 1 | 4-$CF_3$ |
| I.88 | 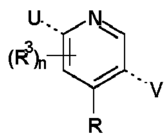 | 0 | - | Cl | H | H | 1 | 4-$CF_3$ |
| I.89 | 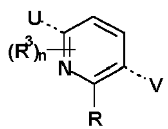 | 0 | - | $CF_3$ | $CH_3$ | H | 2 | 2-F, 4-Cl |
| I.90 | 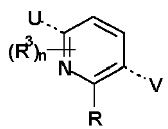 | 0 | - | $CF_3$ | H | H | 2 | 2-F, 4-Cl |

(continued)

| Ex N° | Y | n | $R^3$ | R | $R^1$ | $R^2$ | m | $R^4$ |
|---|---|---|---|---|---|---|---|---|
| I.91 | 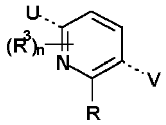 | 0 | - | $CF_3$ | cyclopropyl | H | 1 | 4-Cl |
| I.92 (*) | 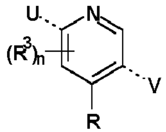 | 0 | - | $CF_3$ | H | H | 1 | 4-$CF_3$ |
| I.93 | 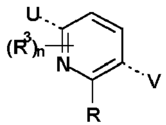 | 0 | - | $CF_3$ | cyclopropyl | H | 1 | 4-$CHF_2$ |
| I.94 | 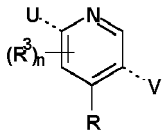 | 0 | - | $CHF_2$ | H | H | 1 | 4-Cl |
| I.95 | 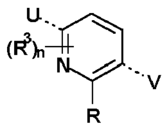 | 0 | - | $CF_3$ | $CH_3$ | H | 1 | 4-$OCF_3$ |
| I.96 | 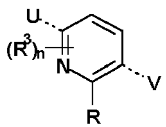 | 0 | - | $CF_3$ | $CH_3$ | H | 1 | 4-Cl |
| I.97 | 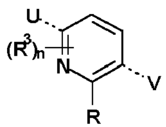 | 0 | - | $CF_3$ | cyclopropyl | H | 1 | 4-$SF_5$ |

(continued)

| Ex N° | Y | n | $R^3$ | R | $R^1$ | $R^2$ | m | $R^4$ |
|---|---|---|---|---|---|---|---|---|
| I.98 | 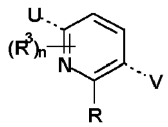 | 0 | - | $CF_3$ | butyl | H | 1 | 4-Cl |
| I.99 (*) | 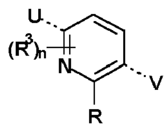 | 0 | - | $CF_3$ | cyclopropyl | H | 1 | 4-Br |
| I.100 | 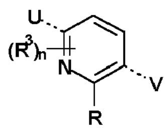 | 0 | - | $CF_3$ | propyl | H | 1 | 4-Cl |
| I.101 (*) | 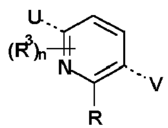 | 0 | - | $CF_3$ | $CH_3$ | H | 1 | 4-Br |
| I.102 (*) | 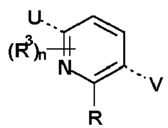 | 0 | - | $CF_3$ | $CH_3$ | H | 1 | 4-Br |
| I.103 | 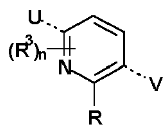 | 0 | - | $CF_3$ | Et | H | 1 | 4-Cl |
| I.104 | 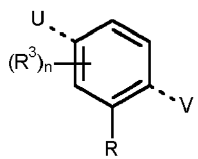 | 0 | - | $CF_3$ | Me | H | 1 | 4-Cl |

(continued)

| Ex N° | Y | n | $R^3$ | R | $R^1$ | $R^2$ | m | $R^4$ |
|---|---|---|---|---|---|---|---|---|
| I.105 | 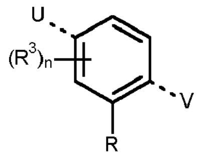 | 0 | - | $CF_3$ | iso-propyl | H | 1 | 4-Cl |
| I.106 | 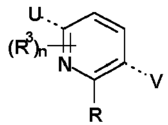 | 0 | - | $CF_3$ | H | H | 1 | 4-CHO |
| I.107 | 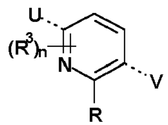 | 0 | - | $CF_3$ | H | H | 1 | 4-S($CH_3$) |
| I.108 | 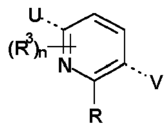 | 0 | - | $CF_3$ | $CH_3$ | H | 1 | 4-S($CH_3$) |
| I.109 | 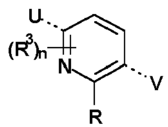 | 0 | - | $CF_3$ | H | H | 1 | 4-hydroxy-iminomethyl |
| I.110 | 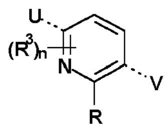 | 0 | - | $CF_3$ | $CH_3$ | H | 1 | 4-cyclopropyl |
| I.111 | 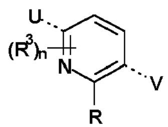 | 0 | - | $CF_3$ | H | H | 1 | 4-cyclopropyl |

(continued)

| Ex N° | Y | n | $R^3$ | R | $R^1$ | $R^2$ | m | $R^4$ |
|---|---|---|---|---|---|---|---|---|
| I.112 | 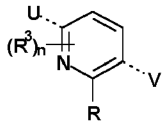 | 0 | - | $CF_3$ | $CH_3$ | H | 1 | 4-$CH_3$ |

Optical rotation

Concentration c is expressed in g/100 mL

(*) Ex 1.45 and 1.46 are the 2 enantiomers of Ex 1.02

(*) Ex 1.53 and 1.54 are the 2 enantiomers of Ex 1.08

Ex 1.53: Optical rotation: -35° (c=0.52, DCM, 20°C)

Ex 1.54: Optical rotation: +52° (c=0.50, DCM, 20°C)

(*) Ex 1.51 and 1.52 are the 2 enantiomers of Ex 1.09

Ex 1.51: Optical rotation: -128.2° (c=0.52, DCM, 20°C)

Ex 1.52: Optical rotation: +133.3° (c=0.51, DCM, 20°C)

(*) Ex 1.83 and 1.86 are the 2 enantiomers of Ex 1.36

Ex 1.83: Optical rotation: +10.0°(c=0.50, $CDCl_3$, 25°C)

Ex 1.86: Optical rotation: -11.0°(c=0.73, $CDCl_3$, 25°C)

(*) Ex 1.72 and 1.92 are the 2 enantiomers of Ex 1.47

Ex 1.72: Optical rotation: +11.7°(c=0.52, $CDCl_3$, 25°C)

Ex 1.92: Optical rotation: -10.4°(c=0.58, $CDCl_3$, 25°C)

(*) Ex I.101 and I.102 are the 2 enantiomers of Ex 1.59

Ex I.101: Optical rotation: +27.5° (c=0.88; MeOH; 20°C)

Ex 1.102: Optical rotation: -31.5° (c=1.02; MeOH; 20°C)

(*) Ex 1.84 and 1.99 are the 2 enantiomers of Ex 1.81

Ex 1.84: Optical rotation: -8°(c=1.00, MeOH, 25°C)

Ex 1.99: Optical rotation: +7.3°(c=1.10, MeOH, 25°C)

In preferred embodiments of the present invention compounds of formula (I) are present, wherein

$R^1$ represents hydrogen, $C_1$-$C_4$-alkyl, or cyclopropyl;

$R^2$ represents hydrogen;

$R^4$ represents $CF_3$, $OCF_3$, Br, Cl or pentafluoro-$\lambda^6$-sulfanyl;

m is 1;

Y represents

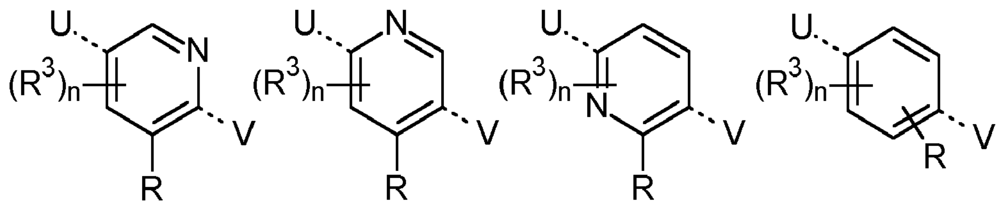

wherein Y is connected to the O of formula (I) via the bonds identified with "U" and Y is connected to the $CR^1(OR^2)$ moiety of formula (I) via the bonds identified with "V" and

R represents $C_1$-haloalkyl; and

n is 0.

In more preferred embodiments of the present invention compounds of formula (I) are present, wherein

$R^1$ represents methyl or cyclopropyl;

$R^2$ represents hydrogen;

$R^4$ represents Cl or Br in the 4-position of the phenyl moiety of formula (I);

m is 1;

Y represents

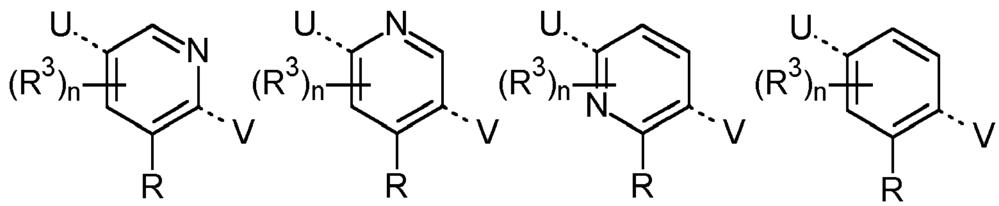

wherein Y is connected to the O of formula (I) via the bonds identified with "U" and Y is connected to the $CR^1(OR^2)$ moiety of formula (I) via the bonds identified with "V" and

R represents $CF_3$; and

n is 0.

More preferred compound combinations according to the invention comprise (A) a compound of formula (I) selected from the group consisting of (1.01) 2-[6-(4-chlorophenoxy)-2-(trifluoromethyl)pyridin-3-yl]-1-(1H-1,2,4-triazol-1-yl)propan-2-ol, (1.59) 2-[6-(4-bromophenoxy)-2-(trifluoromethyl)pyridin-3-yl]-1-(1H-1,2,4-triazol-1-yl)propan-2-ol, (1.81) 1-[6-(4-bromophenoxy)-2-(trifluoromethyl)pyridin-3-yl]-1-cyclopropyl-2-(1H-1,2,4-triazol-1-yl)ethanol, (1.91)

1-[6-(4-chlorophenoxy)-2-(trifluoromethyl)pyridin-3 - yl]-1-cyclopropyl-2-(1H-1,2,4-triazol-1-yl)ethanol, (1.104) 2-[4-(4-chlorophenoxy)-2-(trifluoromethyl)phenyl]-1-(1,2,4-triazol-1-yl)propan-2-ol and (1.105) 2-[4-(4-chlorophenoxy)-2-(trifluoromethyl)phenyl]-3-methyl-1-(1,2,4-triazol-1-yl)butan-2-ol.

Even more preferred compound combinations according to the invention comprise (A) as compound of formula (I) (1.01) 2-[6-(4-chlorophenoxy)-2-(trifluoromethyl)pyridin-3-yl]-1-(1H-1,2,4-triazol-1-yl)propan-2-ol and/or (1.59) 2-[6-(4-bromophenoxy)-2-(trifluoromethyl)pyridin-3-yl]-1-(1H-1,2,4-triazol-1-yl)propan-2-ol and/or (1.104) 2-[4-(4-chlorophenoxy)-2-(trifluoromethyl)phenyl]-1-(1,2,4-triazol-1-yl)propan-2-ol.

Even more preferred compound combinations according to the invention comprise (A) as compound of formula (I) (1.01) 2-[6-(4-chlorophenoxy)-2-(trifluoromethyl)pyridin-3-yl]-1-(1H-1,2,4-triazol-1-yl)propan-2-ol and/or (1.59) 2-[6-(4-bromophenoxy)-2-(trifluoromethyl)pyridin-3-yl]-1-(1H -1,2,4-triazol-1-yl)propan-2-ol.

Most preferred compound combinations according to the invention comprise (A) as compound of formula (I) (I.59) 2-[6-(4-bromophenoxy)-2-(trifluoromethyl)pyridin-3-yl]-1-(1H-1,2,4-triazol-1-yl)propan-2-ol.

In the definitions of the symbols given in the above formulae, collective terms were used which are generally representative of the following substituents:

**Halogen:** fluorine, chlorine, bromine or iodine, preferably fluorine, chlorine or bromine. Halogen-substitution is generally indicated by the prefix halo, halogen or halogeno.

**Alkyl:** saturated, straight-chain or branched hydrocarbyl radical having 1 to 8, preferably 1 to 6, and more preferably 1 to 4 carbon atoms, for example (but not limited to) $C_1$-$C_6$-alkyl such as methyl, ethyl, propyl (n-propyl), 1-methylethyl (iso-propyl), butyl (n-butyl), 1-methylpropyl (sec-butyl), 2-methylpropyl (iso-butyl), 1,1-dimethylethyl (tert-butyl), pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Particularly, said group is a $C_1$-$C_4$-alkyl group, e.g. a methyl, ethyl, propyl, 1-methylethyl (isopropyl), butyl, 1-methylpropyl (sec-butyl), 2-methylpropyl (iso-butyl) or 1,1-dimethylethyl (tert-butyl) group. This definition also applies to alkyl as part of a composite substituent, for example cycloalkylalkyl, hydroxyalkyl etc., unless defined elsewhere like, for example, alkylsulfanyl, alkylsulfinyl, alkylsulfonyl, haloalkyl or haloalkylsulfanyl.

**Alkenyl:** unsaturated, straight-chain or branched hydrocarbyl radicals having 2 to 8, preferably 2 to 6, and more preferably 2 to 4 carbon atoms and one double bond in any position, for example (but not limited to) $C_2$-$C_6$-alkenyl such as vinyl, allyl, (E)-2-methylvinyl, (Z)-2-methylvinyl, isopropenyl, homoallyl, (E)-but-2-enyl, (Z)-but-2-enyl, (E)-but-1-enyl, (Z)-but-1-enyl, 2-methylprop-2-enyl, 1-methylprop-2-enyl, 2-methylprop-1-enyl, (E)-1-methylprop-1-enyl, (Z)-1-methylprop-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, 3- methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1- methylbut-2-enyl, (E)-3-methylbut-1-enyl, (Z)-3-methylbut-1-enyl, (E)-2- methylbut-1-enyl, (Z)-2-methylbut-1-enyl, (E)-1-methylbut-1-enyl, (Z)-1- methylbut-1-enyl, 1,1-dimethylprop-2-enyl, 1-ethylprop-1-enyl, 1-propylvinyl, 1- isopropylvinyl, (E)-3,3-dimethylprop-1-enyl, (Z)-3,3-dimethylprop-1-enyl, hex-5-enyl, (E)-hex-4- enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, 4-methylpent-4-enyl, 3-methylpent-4-enyl, 2-methylpent-4-enyl, 1- methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4- methylpent-2-enyl, (E)-3-methylpent-2-enyl, (Z)-3-methylpent-2-enyl, (E)-2- methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3- methylpent-1-enyl, (Z)-3-methylpent-1 -enyl, (E)-2-methylpent-1-enyl, (Z)-2- methylpent-1-enyl, (E)-1-methylpent-1-enyl, (Z)-1-methylpent-1-enyl, 3-ethylbut- 3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2-enyl, 2-isopropylprop-2-enyl, 1 -isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropylprop-1-enyl, (Z)-2-isopropylprop-1-enyl, (E)-1-isopropylprop-1-enyl, (Z)-1-isopropylprop-1-enyl, 1-(1,1-dimethylethyl)ethenyl, buta-1,3-dienyl, penta-1,4-dienyl, hexa-1,5-dienyl or methylhexadienyl. Particularly, said group is vinyl or allyl. This definition also applies to alkenyl as part of a composite substituent, for example haloalkenyl etc., unless defined elsewhere.

**Alkynyl:** straight-chain or branched hydrocarbyl groups having 2 to 8, preferably 2 to 6, and more preferably 2 to 4 carbon atoms and one triple bond in any position, for example (but not limited to) $C_2$-$C_6$-alkynyl, such as ethynyl, prop-1-ynyl, prop-2-ynyl, but-1-ynyl, but-2-ynyl, but-3-ynyl, 1-methylprop-2-ynyl, pent-1-ynyl, pent-2-ynyl, pent-3-ynyl, pent-4-ynyl, 2-methylbut-3-ynyl, 1 -methylbut-3-ynyl, 1-methylbut-2-ynyl, 3-methylbut-1-ynyl, 1-ethylprop-2-ynyl, hex-1-ynyl, hex-2-ynyl, hex-3-ynyl, hex-4-ynyl, hex-5-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-ynyl. This definition also applies to alkynyl as part of a composite substituent, for example haloalkynyl etc., unless defined elsewhere.

**Alkoxy:** saturated, straight-chain or branched alkoxy radicals having 1 to 8, preferably 1 to 6 and more preferably 1 to 4 carbon atoms, for example (but not limited to) $C_1$-$C_6$-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, 1,1-dimethylpropoxy, 1,2-dimethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. This definition also applies to alkoxy as part of a composite substituent, for example haloalkoxy, alkynylalkoxy, etc., unless defined elsewhere.

**Alkoxycarbonyl:** an alkoxy group which has 1 to 8, preferably 1 to 6 and more preferably 1 to 4 carbon atoms (as specified above) and is bonded to the skeleton via a carbonyl group (-C(=O)-). This definition also applies to alkoxycarbonyl as part of a composite substituent, for example cycloalkylalkoxycarbonyl etc., unless defined elsewhere.

**Alkylsulfanyl:** saturated, straight-chain or branched alkylsulfanyl radicals having 1 to 8, preferably 1 to 6 and more preferably 1 to 4 carbon atoms, for example (but not limited to) $C_1$-$C_6$-alkylsulfanyl such as methylsulfanyl, ethylsulfanyl, propylsulfanyl, 1-methylethylsulfanyl, butylsulfanyl, 1-methylpropylsulfanyl, 2-methylpropylsulfanyl, 1,1-dimethylethylsulfanyl, pentylsulfanyl, 1-methylbutylsulfanyl, 2-methylbutylsulfanyl, 3-methylbutylsulfanyl, 2,2-dimethylpropylsulfanyl, 1-ethylpropylsulfanyl, 1,1-dimethylpropylsulfanyl, 1,2-dimethylpropylsulfanyl, hexylsulfanyl, 1-methylpentylsulfanyl, 2-methylpentylsulfanyl, 3-methylpentylsulfanyl, 4-methylpentylsulfanyl, 1,1-dimethylbutylsulfanyl, 1,2-dimethylbutylsulfanyl, 1,3 -dimethylbutylsulfanyl, 2,2-dimethylbutylsulfanyl, 2,3 -dimethylbutylsulfanyl, 3,3-dimethylbutylsulfanyl, 1-ethylbutylsulfanyl, 2-ethylbutylsulfanyl, 1,1,2-trimethylpropylsulfanyl, 1,2,2-trimethylpropylsulfanyl, 1-ethyl-1-methylpropylsulfanyl and 1-ethyl-2-methylpropylsulfanyl. This definition also applies to alkylsulfanyl as part of a composite substituent, for example haloalkylsulfanyl etc., unless defined elsewhere.

**Alkylsulfinyl:** saturated, straight-chain or branched alkylsulfinyl radicals having 1 to 8, preferably 1 to 6 and more preferably 1 to 4 carbon atoms, for example (but not limited to) $C_1$-$C_6$-alkylsulfinyl such as methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, 1,1-dimethylpropylsulfinyl, 1,2-dimethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl. This definition also applies to alkylsulfinyl as part of a composite substituent, for example haloalkylsulfinyl etc., unless defined elsewhere.

**Alkylsulfonyl:** saturated, straight-chain or branched alkylsulfonyl radicals having 1 to 8, preferably 1 to 6 and more preferably 1 to 4 carbon atoms, for example (but not limited to) $C_1$-$C_6$-alkylsulfonyl such as methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 2,2-dimethylpropylsulfonyl, 1-ethylpropylsulfonyl, 1,1-dimethylpropylsulfonyl, 1,2-dimethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3 -dimethylbutylsulfonyl, 2,2-dimethylbutylsulfonyl, 2,3 -dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl. This definition also applies to alkylsulfonyl as part of a composite substituent, for example alkylsulfonylalkyl etc., unless defined elsewhere.

**Monoalkylamino** represents an amino radical having one alkyl residue with 1 to 4 carbon atoms attached to the nitrogen atom. Non-limiting examples include methylamino, ethylamino, n-propylamino, isopropylamino, n-butylamino and tert-butylamino.

**Dialkylamino** represents an amino radical having two independently selected alkyl residues with 1 to 4 carbon atoms each attached to the nitrogen atom. Non-limiting examples include *N,N*-dimethylamino, *N,N*-diethylamino, *N,N*-diisopropylamino, *N*-ethyl-*N*-methylamino, *N*-methyl-*N*-n-propylamino, *N*-iso-propyl-*N*-n-propylamino and *N*-tert-butyl-*N*-methylamino.

**Cycloalkyl:** monocyclic, saturated hydrocarbyl groups having 3 to 10, preferably 3 to 8 and more preferably 3 to 6 carbon ring members, for example (but not limited to) cyclopropyl, cyclopentyl and cyclohexyl. This definition also applies to cycloalkyl as part of a composite substituent, for example cycloalkylalkyl etc., unless defined elsewhere.

**Cycloalkenyl:** monocyclic, partially unsaturated hydrocarbyl groups having 3 to 10, preferably 3 to 8 and more preferably 3 to 6 carbon ring members, for example (but not limited to) cyclopropenyl, cyclopentenyl and cyclohexenyl. This definition also applies to cycloalkenyl as part of a composite substituent, for example cycloalkenylalkyl etc., unless defined elsewhere.

**Cycloalkoxy:** monocyclic, saturated cycloalkyloxy radicals having 3 to 10, preferably 3 to 8 and more preferably 3 to 6 carbon ring members, for example (but not limited to) cyclopropyloxy, cyclopentyloxy and cyclohexyloxy. This definition also applies to cycloalkoxy as part of a composite substituent, for example cycloalkoxyalkyl etc., unless defined elsewhere.

**Haloalkyl:** straight-chain or branched alkyl groups having 1 to 8, preferably 1 to 6 and more preferably 1 to 4 carbon atoms (as specified above), where some or all of the hydrogen atoms in these groups are replaced by halogen atoms as specified above, for example (but not limited to) $C_1$-$C_3$-haloalkyl such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and 1,1,1-trifluoroprop-2-yl. This definition also applies to haloalkyl as part of a composite substituent, for example haloalkylaminoalkyl etc., unless defined elsewhere.

Haloalkenyl and haloalkynyl are defined analogously to haloalkyl except that, instead of alkyl groups, alkenyl and alkynyl groups are present as part of the substituent.

**Haloalkoxy:** straight-chain or branched alkoxy groups having 1 to 8, preferably 1 to 6 and more preferably 1 to 4 carbon atoms (as specified above), where some or all of the hydrogen atoms in these groups are replaced by halogen atoms as specified above, for example (but not limited to) $C_1$-$C_3$-haloalkoxy such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and 1,1,1-trifluoroprop-2-oxy. This definition also applies to haloalkoxy as part of a composite substituent, for example haloalkoxyalkyl etc., unless defined elsewhere.

**Haloalkylsulfanyl:** straight-chain or branched alkylsulfanyl groups having 1 to 8, preferably 1 to 6 and more preferably 1 to 4 carbon atoms (as specified above), where some or all of the hydrogen atoms in these groups are replaced by halogen atoms as specified above, for example (but not limited to) $C_1$-$C_3$-haloalkylsulfanyl such as chloromethylsulfanyl, bromomethylsulfanyl, dichloromethylsulfanyl, trichloromethylsulfanyl, fluoromethylsulfanyl, difluoromethylsulfanyl, trifluoromethylsulfanyl, chlorofluoromethylsulfanyl, dichlorofluoromethylsulfanyl, chlorodifluoromethylsulfanyl, 1-chloro-ethylsulfanyl, 1-bromoethylsulfanyl, 1-fluoroethylsulfanyl, 2-fluoroethylsulfanyl, 2,2-difluoroethyl-sulfanyl, 2,2,2-trifluoroethylsulfanyl, 2-chloro-2-fluoroethylsulfanyl, 2-chloro-2,2-difluoroethylsulfanyl, 2,2-dichloro-2-fluoroethylsulfanyl, 2,2,2-trichloroethylsulfanyl, pentafluoroethylsulfanyl and 1,1,1-trifluoroprop-2-ylsulfanyl. This definition also applies to haloalkylsulfanyl as part of a composite substituent, for example haloalkylsulfanylalkyl etc., unless defined elsewhere.

**Aryl:** mono-, bi- or tricyclic aromatic or partially aromatic group having 6 to 14 carbon atoms, for example (but not limited to) phenyl, naphthyl, tetrahydronapthyl, indenyl and indanyl. The binding to the superordinate general structure can be carried out via any possible ring member of the aryl residue. Aryl is preferably selected from phenyl, 1-naphthyl and 2-naphthyl. Phenyl is particularly preferred.

**Heteroaryl:** 5 or 6-membered cyclic aromatic group containing at least 1, if appropriate also 2, 3, 4 or 5 heteroatoms, wherein the heteroatoms are each selected independently of one another from the group S, N and O, and which group can also be part of a bi- or tricyclic system having up to 14 ring members, wherein the ring system can be formed with one or two further cycloalkyl, cycloalkenyl, heterocyclyl, aryl and/or heteroaryl residues and wherein benzofused 5 or 6-membered heteroaryl groups are preferred. The binding to the superordinate general structure can be carried out via any possible ring member of the heteroaryl residue. Examples of **5-membered heteroaryl groups which are attached to the skeleton via one of the carbon ring members** are fur-2-yl, fur-3-yl, thien-2-yl, thien-3-yl, pyrrol-2-yl, pyrrol-3-yl, isoxazol-3-yl, isoxazol-4-yl, isoxazol-5-yl, isothiazol-3-yl, isothiazol-4-yl, isothiazol-5-yl, pyrazol-3-yl, pyrazol-4-yl, pyrazol-5-yl, oxazol-2-yl, oxazol-4-yl, oxazol-5-yl, thiazol-2-yl, thiazol-4-yl, thiazol-5-yl, imidazol-2-yl, imidazole-4-yl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl. Examples of **5-membered heteroaryl groups which are attached to the skeleton via a nitrogen ring member** are pyrrol-1-yl, pyrazol-1-yl, 1,2,4-triazol-1-yl, imidazol-1-yl, 1,2,3-triazol-1-yl and 1,3,4-triazol-1-yl. Examples of **6-membered heteroaryl groups** are pyridine-2-yl, pyridine-3-yl, pyridine-4-yl, pyridazin-3-yl, pyridazin-4-yl, pyrimidin-2-yl, pyrimidin-4-yl, pyrimidin-5-yl, pyrazine-2-yl, 1,3,5-triazin-2-yl, 1,2,4-triazin-3-yl and 1,2,4,5-tetrazin-3-yl. Examples of **benzofused 5-membered heteroaryl groups** are indol-1-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl and 1,3-benzoxazol-7-yl. Examples of **benzofused 6-membered heteroaryl groups are** quinolin-2-yl, quinolin-3-yl, quinolin-4-yl, quinolin-5-yl,

quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl and isoquinolin-8-yl. Further examples of 5- or 6-membered heteroaryls which are part of a bicyclic ring system are 1,2,3,4-tetrahydroquinolin-1-yl, 1,2,3,4-tetrahydroquinolin-2-yl, 1,2,3,4-tetrahydroquinolin-7-yl, 1,2,3,4-tetrahydroquinolin-8-yl, 1,2,3,4-tetrahydroisoquinolin-1-yl, 1,2,3,4-tetrahydroisoquinolin-2-yl, 1,2,3,4-tetrahydroisoquinolin-5-yl, 1,2,3,4-tetrahydroisoquinolin-6-yl and 1,2,3,4-tetrahydroisoquinolin-7-yl. This definition also applies to heteroaryl as part of a composite substituent, for example heteroarylalkyl etc., unless defined elsewhere.

**Heterocyclyl:** three- to seven-membered, saturated or partially unsaturated heterocyclic group containing at least one, if appropriate up to four heteroatoms and/or heterogroups independently selected from the group consisting of N, O, S, S(=O), $S(=O)_2$ and di-($C_1$-$C_4$)alkylsilyl, which group can be benzofused. The binding to the superordinate general structure can be carried out via a ring carbon atom or, if possible, via a ring nitrogen atom of the heterocyclic group. **Saturated heterocyclic groups** in this sense are for example (but not limited to) oxiranyl, aziridinyl, tetrahydrofuran-2-yl, tetrahydrofuran-3-yl, tetrahydrothien-2-yl, tetrahydrothien-3-yl, pyrrolidin-2-yl, pyrrolidin-3-yl, isoxazolidin-3-yl, isoxazolidin-4-yl, isoxazolidin-5-yl, isothiazolidin-3-yl, isothiazolidin-4-yl, isothiazolidin-5-yl, pyrazolidin-3-yl, pyrazolidin-4-yl, pyrazolidin-5-yl, oxazolidin-2-yl, oxazolidin-4-yl, oxazolidin-5-yl, thiazolidin-2-yl, thiazolidin-4-yl, thiazolidin-5-yl, imidazolidin-2-yl, imidazolidin-4-yl, 1,2,4-oxadiazolidin-3-yl, 1,2,4-oxadiazolidin-5-yl, 1,3,4-oxadiazolidin-2-yl, 1,2,4-thiadiazolidin-3-yl, 1,2,4-thiadiazolidin-5-yl, 1,3,4-thiadiazolidin-2-yl, 1,2,4-triazolidin-3-yl, 1,3,4-triazolidin-2-yl, piperidin-2-yl, piperidin-3-yl, piperidin-4-yl, 1,3-dioxan-5-yl, tetrahydropyran-2-yl, tetrahydropyran-4-yl, tetrahydrothien-2-yl, hexahydropyridazin-3-yl, hexa-hydropyridazin-4-yl, hexahydropyrimidin-2-yl, hexahydropyrimidin-4-yl, hexahydropyrimidin-5-yl, piperazin-2-yl, 1,3,5-hexahydrotriazin-2-yl and 1,2,4-hexahydrotriazin-3-yl. **Partially unsaturated heterocyclic groups** in this sense are for example (but not limited to) 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl. Examples of **benzofused heterocyclic groups** are indolin-1-yl, indolin-2-yl, indolin-3-yl, isoindolin-1-yl, isoindolin-2-yl, 2,3-dihydrobenzofuran-2-yl and 2,3-dihydrobenzofuran-3-yl. This definition also applies to heterocyclyl as part of a composite substituent, for example heterocyclylalkyl etc., unless defined elsewhere.

Oxo represents a doubly bonded oxygen atom.

**Thiooxo** represents a doubly bonded sulfur atom.

Optionally substituted groups may be mono- or polysubstituted, where the substituents in the case of polysubstitutions may be identical or different.

Not included are combinations which are against natural laws and which the person skilled in the art would therefore exclude based on his/her expert knowledge. Ring structures having three or more adjacent oxygen atoms, for example, are excluded.

Compounds of formula (I) are known and can be obtained by various prior art processes. Compounds of formula (I), wherein Y is selected from

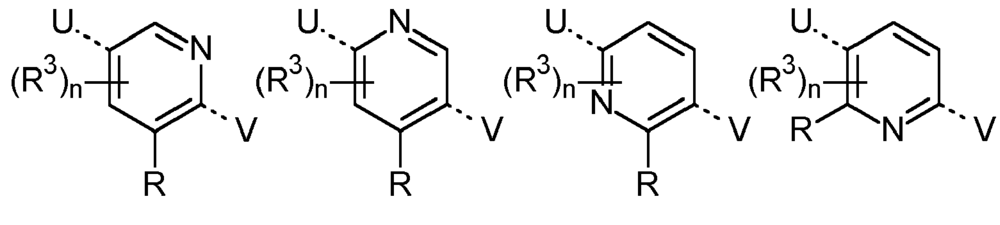

,

their use to control fungal diseases of plants and their preparation are for example disclosed in WO-A 2017/029179. Compounds of formula **(I),** wherein Y is

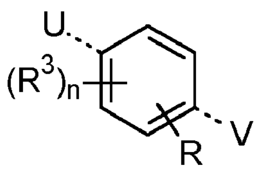

their use to control fungal diseases of plants and their preparation are for example disclosed in WO-A 2013/007767.

*Isomers*

Depending on the nature of the substituents, compounds present in the compound combinations of the invention may be present in the form of different stereoisomers. These stereoisomers are, for example, enantiomers, diastereomers, atropisomers or geometric isomers. Accordingly, the invention encompasses both pure stereoisomers and any mixture of these isomers. Where a compound can be present in two or more tautomer forms in equilibrium, reference to the compound by means of one tautomeric description is to be considered to include all tautomer forms.
The compounds of formula (I) as well as florylpicoxamid can be converted into physiologically acceptable salts, e.g. as acid addition salts or metal salt complexes.
Depending on the nature of the substituents defined above, the compounds have acidic or basic properties and can form salts, if appropriate also inner salts, or adducts with inorganic or organic acids or with bases or with metal ions. If the compounds carry amino, alkylamino or other groups which induce basic properties, these compounds can be reacted with acids to give salts, or they are directly obtained as salts in the synthesis. If the compounds carry hydroxyl, carboxyl or other groups which induce acidic properties, these compounds can be reacted with bases to give salts. Suitable bases are, for example, hydroxides, carbonates, bicarbonates of the alkali metals and alkaline earth metals, in particular those of sodium, potassium, magnesium and calcium, furthermore ammonia, primary, secondary and tertiary amines having ($C_1$-$C_4$)-alkyl groups, mono-, di- and trialkanolamines of ($C_1$-$C_4$)-alkanols, choline and also chlorocholine.
The salts obtainable in this manner also have fungicidal properties.
Examples of inorganic acids are hydrohalic acids, such as hydrogen fluoride, hydrogen chloride, hydrogen bromide and hydrogen iodide, sulphuric acid, phosphoric acid and nitric acid, and acidic salts, such as $NaHSO_4$ and $KHSO_4$. Suitable organic acids are, for example, formic acid, carbonic acid and alkanoic acids, such as acetic acid, trifluoroacetic acid, trichloroacetic acid and propionic acid, and also glycolic acid, thiocyanic acid, lactic acid, succinic acid, citric acid, benzoic acid, cinnamic acid, maleic acid, fumaric acid, tartaric acid, sorbic acid oxalic acid, alkylsulphonic acids (sulphonic acids having straight-chain or branched alkyl radicals of 1 to 20 carbon atoms), arylsulphonic acids or aryldisulphonic acids (aromatic radicals, such as phenyl and naphthyl, which carry one or two sulphonic acid groups), alkylphosphonic acids (phosphonic acids having straight-chain or branched alkyl radicals of 1 to 20 carbon atoms), arylphosphonic acids or aryldiphosphonic acids (aromatic radicals, such as phenyl and naphthyl, which carry one or two phosphonic acid radicals), where the alkyl and aryl radicals may carry further substituents, for example p-toluenesulphonic acid, 1,5-naphthalenedisulphonic acid, salicylic acid, p-aminosalicylic acid, 2-phenoxybenzoic acid, 2-acetoxybenzoic acid, etc.
Suitable metal ions are in particular the ions of the elements of the second main group, in particular calcium and magnesium, of the third and fourth main group, in particular aluminium, tin and lead, and also of the first to eighth transition group, in particular chromium, manganese, iron, cobalt, nickel, copper, zinc and others. Particular preference is given to the metal ions of the elements of the fourth period. Here, the metals can be present in various valencies that they can assume.
The acid addition salts can be obtained in a simple manner by customary methods for forming salts, for example by dissolving a respective compound in a suitable inert solvent and adding the acid, for example hydrochloric acid, and be isolated in a known manner, for example by filtration, and, if required, be purified by washing with an inert organic solvent.
Suitable anions of the salts are those which are preferably derived from the following acids: hydrohalic acids, such as, for example, hydrochloric acid and hydrobromic acid, furthermore phosphoric acid, nitric acid and sulphuric acid.
The metal salt complexes can be obtained in a simple manner by customary processes, for example by dissolving the metal salt in alcohol, for example ethanol, and adding the solution to the respective compound. Metal salt complexes can be isolated in a known manner, for example by filtration, and, if required, be purified by recrystallization.
Salts of the intermediates can also be prepared according to the processes mentioned above.
N-oxides of compounds of the formula (I), florylpicoxamid or intermediates thereof can be obtained in a simple manner by customary processes, for example by N-oxidation with hydrogen peroxide ($H_2O_2$), peracids, for example peroxy sulfuric acid or peroxy carboxylic acids, such as meta-chloroperoxybenzoic acid or peroxymonosulfuric acid (Caro's acid).

E.g. the corresponding N-oxides may be prepared starting from compounds **(I)** using conventional oxidation methods, e.g. by treating compounds **(I)** with an organic peracid such as metachloroperbenzoic acid (e.g. WO-A 2003/64572 or J. Med. Chem. 38 (11), 1892-1903, 1995); or with inorganic oxidizing agents such as hydrogen peroxide (e.g. J. Heterocyc. Chem. 18 (7), 1305-1308, 1981) or oxone (e.g. J. Am. Chem. Soc. 123 (25), 5962-5973, 2001). The oxidation may lead to pure mono-N-oxides or to a mixture of different N-oxides, which can be separated by conventional methods such as chromatography.

The active compound combinations according to the invention comprise as component (B) florylpicoxamid.

Florylpicoxamid is the common name of [(1S)-2,2-bis(4-fluorophenyl)-1-methyl-ethyl] (2S)-2-[(3-acetoxy-4-methoxy-pyridine-2-carbonyl)amino]propanoate, also named (1S)-2,2-bis(4-fluorophenyl)-1-methylethyl N-[[3-(acetyloxy)-4-methoxy-2-pyridinyl]carbonyl]-L-alaninate, having the CAS Reg. No: 1961312-55-9. In the following florylpicoxamid is also referred to as compound (II).

Preferred compound combinations are selected from the group (G1) consisting of the following mixtures:
(I.01) + (II), (1.59) + (II), (I.81) + (II), (1.91) + (II), (I.104) + (II), (I.105) + (II), (I.01) + (I.104) + (II), (1.59) + (I.104) + (II), (1.81) + (I.104) + (II), (1.91) + (I.104) + (II).

More preferred compound combinations are selected from the group (G1-A) consisting of the following mixtures:
(I.01) + (II), (1.59) + (II), (I.104) + (II), (I.105) + (II), (1.01) + (I.104) + (II), (1.59) + (I.104) + (II).

Even more preferred compound combinations are selected from the group (G1-B) consisting of the following mixtures:
(I.01) + (II), (I.59) + (II), (I.104) + (II).

Most preferred the compound combinations are selected from the group (G1-C) consisting of the following mixtures:
(I.01) + (II), (1.59) + (II).

In the combinations according to the invention the compounds (A), i.e. triazole derivatives of formula (I), and (B), i.e. florylpicoxamid (II), can be present in a broad range of effective weight ratio of A:B, for example in a range of 100:1 to 1:100, preferably in a weight ratio of 50:1 to 1:50, most preferably in a weight ratio of 20:1 to 1:20. Further ratios of A:B which can be used according to the present invention with increasing preference in the order given are: 95:1 to 1:95, 90:1 to 1:90, 85:1 to 1:85, 80:1 to 1:80, 75:1 to 1:75, 70:1 to 1:70, 65:1 to 1:65, 60:1 to 1:60, 55:1 to 1:55, 45:1 to 1:45, 40:1 to 1:40, 35:1 to 1:35, 30:1 to 1:30,25:1 to 1:25, 15:1 to 1:15, 10:1 to 1:10, 5:1 to 1:5,4:1 to 1:4, 3:1 to 1:3,2:1 to 1:2.

Further ratios of A:B which can be used according to the present invention are: 95:1 to 1:1, 90:1 to 1:1, 85:1 to 1:1, 80:1 to 1:1, 75:1 to 1:1, 70:1 to 1:1, 65:1 to 1:1, 60:1 to 1:1, 55:1 to 1:1, 45:1 to 1:1, 40:1 to 1:1, 35:1 to 1:1, 30:1 to 1:1,25:1 to 1:1, 15:1 to 1:1, 10:1 to 1:1, 5:1 to 1:1,4:1 to 1:1, 3:1 to 1:1,2:1 to 1:1.

Further ratios of A:B which can be used according to the present invention are: 1:1 to 1:95, 1:1 to 1:90, 1:1 to 1:85, 1:1 to 1:80, 1:1 to 1:75, 1:1 to 1:70, 1:1 to 1:65, 1:1 to 1:60, 1:1 to 1:55, 1:1 to 1:45, 1:1 to 1:40, 1:1 to 1:35,1:1 to 1:30, 1:1 to 1:25, 1:1 to 1:15, 1:1 to 1:10, 1:1 to 1:5, 1:1 to 1:4, 1:1 to 1:3, 1:1 to 1:2.

If more than one, e.g. 2 or 3, compounds (A) are present, the weight ratio refers to the total amount of compound (A), i.e. to the sum of the amount of each compound (A) present in the combination.

Where a compound (A) or a compound (B) can be present in isomeric forms and/or tautomeric forms, such a compound is understood hereinabove and hereinbelow also to include, where applicable, corresponding isomeric and/or tautomeric forms or mixtures thereof, even when these are not specifically mentioned in each case.

*Further active compounds*

The active compound combinations according to the invention may comprise as compound (C) at least one further active compound which is different from the compounds of formula (I) and florylpicoxamid and which is selected from the following groups

(1) inhibitors of the ergosterol synthesis,

(2) inhibitors of the respiratory chain at complex I or II,

(3) inhibitors of the respiratory chain at complex III,

(4) inhibitors of the mitosis and cell division,

(5) compounds capable of having a multisite action,

(6) compounds capable of inducing a host defense,

(7) inhibitors of the amino acid and/or protein biosynthesis,

(8) inhibitors of the ATP production,

(9) inhibitors of the cell wall synthesis,

(10) inhibitors of the lipid and membrane synthesis,

(11) inhibitors of the melanine biosynthesis,

(12) inhibitors of the nucleic acid synthesis,

(13) inhibitors of the signal transduction,

(14) compounds capable of acting as uncoupler,

(15) other fungicides.

Such further active compound is preferably selected from:

inhibitors of the ergosterol synthesis selected from the group consisting of (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)-cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)- [3 -(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1 -({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) 2-[4-(4-chlorophenoxy)-2-(trifluoromethyl)phenyl]-1-(1H-1,2,4-triazol-1-yl)propan-2-ol, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{[3-(2,2,3,3-tetrafluoropropoxy)phenyl]sulfanyl}phenyl)-N-ethyl-N-methylimidoformamide, (1.066) N'-(2,5-dimethyl-4- {[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxy]-2-methylpyridin-3-yl}-N-ethyl-N-methylimidoformamide, (1.078) N'- {5-bromo-6-[(cis-4-isopropylcyclohexyl)oxy]-2-methylpyridin-3-yl} -N-ethyl-N-methylimidoformamide, (1.079) N'- {5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide,

inhibitors of the respiratory chain at complex I or II selected from the group consisting of (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3 S)-1,1,3-trimethyl-2,3-dihydro-1H-inden-4-yl]-1H-pyrazole-4-carboxamide, (2.030) 3-(difluoromethyl)-N-(7-fluoro-1,1,3-trimethyl-2,3-dihydro-1H-inden-4-yl)-1-methyl-1H-pyrazole-4-carboxamide, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{[4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, and (2.056) N-cyclopropyl-N-(2-cyclopropylbenzyl)-3-(difluoromethyl)-5-fluoro-1-methyl-1H-pyrazole-4-carboxamide,

inhibitors of the respiratory chain at complex III selected from the group consisting of (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, and (3.029) methyl {5-[3-(2,4-dimethylphenyl)-1H-pyrazol-1-yl]-2-methylbenzyl}carbamate,

inhibitors of the mitosis and cell division selected from the group consisting of (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, and (4.025) N-(4-chloro-2,6-difluorophenyl)-4-(2-chloro-4-fluorophenyl)-1,3-dimethyl-1H-pyrazol-5-amine,

compounds capable of having a multisite action selected from the group consisting of (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, and (5.023) 6-ethyl-5,7-dioxo-6,7-dihydro-5H-pyrrolo[3',4':5,6][1,4]dithiino[2,3-c][1,2]thiazole-3-carbonitrile,

compounds capable of inducing a host defense selected from the group consisting of (6.001) acibenzolar-S-methyl, (6.002) isotianil, (6.003) probenazole, and (6.004) tiadinil,

inhibitors of the amino acid and/or protein biosynthesis selected from the group consisting of (7.001) cyprodinil, (7.002) kasugamycin, (7.003) kasugamycin hydrochloride hydrate, (7.004) oxytetracycline, (7.005) pyrimethanil, and (7.006) 3-(5-fluoro-3,3,4,4-tetramethyl-3,4-dihydroisoquinolin-1-yl)quinolone,

inhibitors of the ATP production selected from the group consisting of (8.001) silthiofam,

inhibitors of the cell wall synthesis selected from the group consisting of (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, and (9.009) (2Z)-3-(4-tert-butylphenyl)-3-(2-chloropyridin-4-yl)-1-(morpholin-4-yl)prop-2-en-1-one,

inhibitors of the lipid and membrane synthesis selected from the group consisting of (10.001) propamocarb, (10.002) propamocarb hydrochloride, and (10.003) tolclofos-methyl,

inhibitors of the melanine biosynthesis selected from the group consisting of (11.001) tricyclazole, and (11.002) 2,2,2-trifluoroethyl {3-methyl-1-[(4-methylbenzoyl)amino]butan-2-yl}carbamate,

inhibitors of the nucleic acid synthesis selected from the group consisting of (12.001) benalaxyl, (12.002) benalaxyl-M (kiralaxyl), (12.003) metalaxyl, and (12.004) metalaxyl-M (mefenoxam),

inhibitors of the signal transduction selected from the group consisting of (13.001) fludioxonil, (13.002) iprodione, (13.003) procymidone, (13.004) proquinazid, (13.005) quinoxyfen, and (13.006) vinclozolin,

compounds capable of acting as uncoupler selected from the group consisting of (14.001) fluazinam, and (14.002) meptyldinocap,

other fungicides selected from the group consisting of (15.001) abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) 2,6-dimethyl-1H,5H-[1,4]dithiino[2,3-c:5,6-c']dipyrrole-1,3,5,7(2H,6H)-tetrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4- {5- [2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl} -3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) 2-{2-[(7,8-difluoro-2-methylquinolin-3-yl)oxy]-6-fluorophenyl}propan-2-ol, (15.042) 2-{2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl} -3 -chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) 3-(4,4-difluoro-3,3-dimethyl-3,4-dihydroisoquinolin-1-yl)quinoline, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, and (15.062) 5-fluoro-4-imino-3-methyl-1-[(4-methylphenyl)sulfonyl]-3,4-dihydropyrimidin-2(1H)-one.

Such further active compound is more preferred selected from:

(1.001) cyproconazole, (1.002) difenoconazole, (1.003) epoxiconazole, (1.004) fenhexamid, (1.010) imazalil, (1.012) ipconazole, (1.013) metconazole, (1.017) propiconazole, (1.018) prothioconazole, (1.020) spiroxamine, (1.021) tebuconazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.059) 5-(4-chlorobenzyl)-2-(chloromethyl)-2-methyl-1-(1H-1,2,4-triazol-1-ylmethyl)cyclopentanol,

(2.001) benzovindiflupyr, (2.002) bixafen, (2.003) boscalid, (2.005) fluopyram, (2.007) fluxapyroxad, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.021) sedaxane, (2.027) 3-(difluoromethyl)-1-methyl-N-(1,1,3-trimethyl-2,3-dihydro-1H-inden-4-yl)-1H-pyrazole-4-carboxamide, (2.030) 3-(difluoromethyl)-N-(7-fluoro-1,1,3-trimethyl-2,3-dihydro-1H-inden-4-yl)-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.003) azoxystrobin, (3.007) dimoxystrobin, (3.012) fluoxastrobin, (3.013) kresoxim-methyl, (3.016) picoxystrobin, (3.017) pyraclostrobin, (3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide,

(4.005) pencycuron, (4.007) thiophanate-methyl, (4.012) 4-(2-bromo-4-fluorophenyl)-N-(2,6-difluorophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine,

(5.003) captan, (5.004) chlorothalonil, (5.011) dodine, (5.012) folpet, (5.013) mancozeb, (5.015) metiram, (5.018) propineb,

(6.002) isotianil,

(7.001) cyprodinil, (7.005) pyrimethanil,

(12.003) metalaxyl, (12.004) metalaxyl-M (mefenoxam),

(13.001) fludioxonil, (13.002) iprodione, (13.004) proquinazid, (13.005) quinoxyfen,

(14.001) fluazinam, (14.002) meptyldinocap,

(15.008) cyflufenamid, (15.010) cyprosulfamide, (15.011) flutianil, (15.012) fosetyl-aluminium, (15.016) metrafenone, (15.027) pyriofenone (chlazafenone), and (15.047) 3-(4,4-difluoro-3,3-dimethyl-3,4-dihydroisoquinolin-1-yl)quinolone, (15.048) 4-amino-5-fluoropyrimidin-2-ol (tautomeric form: 4-amino-5-fluoropyrimidin-2(1H)-one), (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.062) 5-fluoro-4-imino-3-methyl-1-[(4-methylphenyl)sulfonyl]-3,4-dihydropyrimidin-2(1H)-one.

Such further active compound is even more preferred selected from:

(1.002) difenoconazole, (1.010) imazalil, (1.012) ipconazole, (1.018) prothioconazole, (1.020) spiroxamine, (1.021) tebuconazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.059) 5-(4-chlorobenzyl)-2-(chloromethyl)-2-methyl-1-(1H-1,2,4-triazol-1-ylmethyl)cyclopentanol,

(2.001) benzovindiflupyr, (2.002) bixafen, (2.005) fluopyram, (2.007) fluxapyroxad, (2.017) penflufen, (2.018) penthiopyrad, (2.019) pydiflumetofen, (2.021) sedaxane, (2.027) 3-(difluoromethyl)-1-methyl-N-(1,1,3-trimethyl-2,3-dihydro-1H-inden-4-yl)-1H-pyrazole-4-carboxamide, (2.030) 3-(difluoromethyl)-N-(7-fluoro-1,1,3-trimethyl-2,3-dihydro-1H-inden-4-yl)-1 -methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.003) azoxystrobin, (3.012) fluoxastrobin, (3.016) picoxystrobin, (3.017) pyraclostrobin, (3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide,

(4.005) pencycuron, (4.007) thiophanate-methyl, (4.012) 4-(2-bromo-4-fluorophenyl)-N-(2,6-difluorophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine,

(5.004) chlorothalonil, (5.011) dodine, (5.012) folpet, (5.013) mancozeb, (5.018) propineb,

(6.002) isotianil,

(7.005) pyrimethanil,

(12.003) metalaxyl, (12.004) metalaxyl-M (mefenoxam),

(13.001) fludioxonil, (13.004) proquinazid,

(14.001) fluazinam, (14.002) meptyldinocap,

(15.008) cyflufenamid, (15.027) pyriofenone (chlazafenone), (15.047) 3-(4,4-difluoro-3,3-dimethyl-3,4-dihydroisoquinolin-1-yl)quinolone, (15.048) 4-amino-5-fluoropyrimidin-2-ol (tautomeric form: 4-amino-5-fluoropyrimidin-2(1H)-one), (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.062) 5-fluoro-4-imino-3-methyl-1-[(4-methylphenyl)sulfonyl]-3,4-dihydropyrimidin-2(1H)-one.

Such further active compound is more preferred selected from:

(1.012) ipconazole, (1.018) prothioconazole, (1.020) spiroxamine, (1.021) tebuconazole,

(2.002) bixafen, (2.005) fluopyram, (2.017) penflufen, (2.027) 3-(difluoromethyl)-1-methyl-N-(1,1,3-trimethyl-2,3-dihydro-1H-inden-4-yl)-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate,

(4.005) pencycuron,

(5.004) chlorothalonil, (5.013) mancozeb, (5.018) propineb,

(12.003) metalaxyl, (12.004) metalaxyl-M (mefenoxam),

(13.001) fludioxonil, (13.004) proquinazid,

(15.008) cyflufenamid, and (15.047) 3-(4,4-difluoro-3,3-dimethyl-3,4-dihydroisoquinolin-1-yl)quinoline.

Such further active compound is most preferred selected from:
(2.002) bixafen, (2.005) fluopyram, (2.017) penflufen, (2.027) 3-(difluoromethyl)-1-methyl-N-(1,1,3-trimethyl-2,3-dihydro-1H-inden-4-yl)-1H-pyrazole-4-carboxamide (common name: inpyrfluxam), (2.038) N-(5-chloro-2-isopropylbenzyl)-N-cyclopropyl-3-(difluoromethyl)-5-fluoro-1-methyl-1H-pyrazole-4-carboxamide (common name: isoflucypram).

Preferred compound combinations comprising at least one compound (C) are selected from the group (T1) consisting of the following mixtures:

(I.01) + (II) + (2.002), (1.59) + (II) + (2.002), (I.81) + (II) + (2.002), (1.91) + (II) + (2.002), (I.104) + (II) + (2.002), (I.105) + (II) + (2.002), (1.01) + (I.104) + (II) + (2.002), (I.59) + (I.104) + (II) + (2.002), (I.81) + (I.104) + (II) + (2.002), (1.91) + (I.104) + (II) + (2.002), (1.01) + (II) + (2.005), (1.59) + (II) + (2.005), (I.81) + (II) + (2.005), (1.91) + (II) + (2.005), (I.104) + (II) + (2.005), (I.105) + (II) + (2.005), (1.01) + (I.104) + (II) + (2.005), (1.59) + (I.104) + (II) + (2.005), (I.81) + (I.104) + (II) + (2.005), (I.91) + (I.104) + (II) + (2.005), (1.01) + (II) + (2.017), (1.59) + (II) + (2.017), (I.81) + (II) + (2.017), (1.91) + (II) + (2.017), (I.104) + (II) + (2.017), (I.105) + (II) + (2.017), (1.01) + (I.104) + (II) + (2.017), (1.59) + (I.104) + (II) + (2.017), (I.81) + (I.104) + (II) + (2.017), (1.91) + (I.104) + (II) + (2.017), (1.01) + (II) + (2.027), (I.59) + (II) + (2.027), (1.81) + (II) + (2.027), (1.91) + (II) + (2.027), (I.104) + (II) + (2.027), (I.105) + (II) + (2.027), (I.01) + (I.104) + (II) + (2.027), (1.59) + (I.104) + (II) + (2.027), (I.81) + (I.104) + (II) + (2.027), (I.91) + (I.104) + (II) + (2.027), (I.01) + (II) + (2.038), (1.59) + (II) + (2.038), (I.81) + (II) + (2.038), (I.91) + (II) + (2.038), (I.104) + (II) + (2.038), (I.105) + (II) + (2.038), (I.01) + (I.104) + (II) + (2.038), (1.59) + (I.104) + (II) + (2.038), (1.81) + (I.104) + (II) + (2.038), (I.91) + (I.104) + (II) + (2.038).

More preferred compound combinations comprising at least one compound (C) are selected from the group (T1-A) consisting of the following mixtures:

(I.01) + (II) + (2.002), (1.59) + (II) + (2.002), (I.104) + (II) + (2.002), (I.105) + (II) + (2.002), (1.01) + (I.104) + (II) + (2.002), (1.59) + (I.104) + (II) + (2.002), (1.01) + (II) + (2.005), (1.59) + (II) + (2.005), (I.104) + (II) + (2.005), (I.105) + (II) + (2.005), (1.01) + (I.104) + (II) + (2.005), (1.59) + (I.104) + (II) + (2.005), (1.01) + (II) + (2.017), (1.59) + (II) + (2.017), (I.104) + (II) + (2.017), (I.105) + (II) + (2.017), (I.01) + (I.104) + (II) + (2.017), (1.59) + (I.104) + (II) + (2.017), (1.01) + (II) + (2.027), (1.59) + (II) + (2.027), (I.104) + (II) + (2.027), (I.105) + (II) + (2.027), (1.01) + (I.104) + (II) + (2.027), (1.59) + (I.104) + (II) + (2.027), (1.01) + (II) + (2.038), (1.59) + (II) + (2.038), (I.104) + (II) + (2.038), (I.105) + (II) + (2.038), (I.01) + (I.104) + (II) + (2.038), (1.59) + (I.104) + (II) + (2.038).

Even more preferred compound combinations are selected from the group (T1-B) consisting of the following mixtures:

(I.01) + (II) + (2.002), (1.59) + (II) + (2.002), (I.104) + (II) + (2.002), (1.01) + (II) + (2.005), (1.59) + (II) + (2.005), (I.104) + (II) + (2.005), (1.01) + (II) + (2.017), (1.59) + (II) + (2.017), (I.104) + (II) + (2.017), (I.01) + (II) + (2.027), (1.59) + (II) + (2.027), (I.104) + (II) + (2.027), (1.01) + (II) + (2.038), (1.59) + (II) + (2.038), (I.104) + (II) + (2.038).

Most preferred the compound combinations are selected from the group (T1-C) consisting of the following mixtures:

(I.01) + (II) + (2.002), (1.59) + (II) + (2.002), (1.01) + (II) + (2.005), (1.59) + (II) + (2.005), (1.01) + (II) + (2.017), (1.59) + (II) + (2.017), (1.01) + (II) + (2.027), (1.59) + (II) + (2.027), (1.01) + (II) + (2.038), (1.59) + (II) + (2.038).

In the combinations according to the invention comprising besides at least one compound (A), i.e. a triazole compound of formula (I), and the compound (B), i.e. florylpicoxamid (II), at least one further compound (C), i.e. a further active compound selected from groups (1) to (15) as outlined above, the weight ratio of A:C can vary in a broad range, for example in a range of 100:1 to 1:100, preferably in a weight ratio of 50:1 to 1:50, most preferably in a weight ratio of 20:1 to 1:20. Further ratios of A:C which can be used according to the present invention with increasing preference in the order given are: 95:1 to 1:95, 90:1 to 1:90, 85:1 to 1:85, 80:1 to 1:80, 75:1 to 1:75, 70:1 to 1:70, 65:1 to 1:65, 60:1 to 1:60, 55:1 to 1:55, 45:1 to 1:45, 40:1 to 1:40, 35:1 to 1:35, 30:1 to 1:30, 25:1 to 1:25, 15:1 to 1:15, 10:1 to 1:10, 5:1 to 1:5, 4:1 to 1:4, 3:1 to 1:3, 2:1 to 1:2.

Further ratios of A:C which can be used according to the present invention are: 95:1 to 1:1, 90:1 to 1:1, 85:1 to 1:1, 80:1 to 1:1, 75:1 to 1:1, 70:1 to 1:1, 65:1 to 1:1, 60:1 to 1:1, 55:1 to 1:1, 45:1 to 1:1, 40:1 to 1:1, 35:1 to 1:1, 30:1 to 1:1, 25:1 to 1:1, 15:1 to 1:1, 10:1 to 1:1, 5:1 to 1:1,4:1 to 1:1, 3:1 to 1:1,2:1 to 1:1.

Further ratios of A:C which can be used according to the present invention are: 1:1 to 1:95, 1:1 to 1:90, 1:1 to 1:85, 1:1 to 1:80, 1:1 to 1:75, 1:1 to 1:70, 1:1 to 1:65, 1:1 to 1:60, 1:1 to 1:55, 1:1 to 1:45, 1:1 to 1:40, 1:1 to 1:35,1:1 to 1:30, 1:1 to 1:25, 1:1 to 1:15, 1:1 to 1:10, 1:1 to 1:5, 1:1 to 1:4, 1:1 to 1:3, 1:1 to 1:2.

If more than one, e.g. 2 or 3, compounds (A) are present, the weight ratio refers to the total amount of compound (A), i.e. to the sum of the amount of each compound (A) present in the combination. This applies mutatis mutandis, if more than one, e.g. 2 or 3, compounds (C) are present, i.e. in such case the weight ratio refers to the total amount of compound (C), i.e. to the sum of the amount of each compound (C) present in the combination.

*Methods and uses*

The invention also relates to a method for controlling unwanted microorganisms, characterized in that a compound combination according to the invention or a composition comprising such combination is applied to the microorganisms and/or in their habitat.

The invention further relates to seed which has been treated with a compound combination according to the invention or a composition comprising such combination.

The invention finally provides a method for protecting seed against unwanted microorganisms by using seed treated with a compound combination according to the invention or a composition comprising such combination.

The compound combinations according to the invention and compositions comprising such combination have

potent microbicidal activity and can be used for control of unwanted microorganisms, such as fungi and bacteria, in crop protection and in the protection of materials.

The compound combinations according to the invention and compositions comprising such combination have very good fungicidal properties and can be used in crop protection, for example for control of Plasmodiophoromycetes, Oomycetes, Chytridiomycetes, Zygomycetes, Ascomycetes, Basidiomycetes and Deuteromycetes.

Bactericides can be used in crop protection, for example, for control of Pseudomonadaceae, Rhizobiaceae, Enterobacteriaceae, Corynebacteriaceae and Streptomycetaceae.

The compound combinations according to the invention and compositions comprising such combination can be used for curative or protective control of phytopathogenic fungi. The invention therefore also relates to curative and protective methods for controlling phytopathogenic fungi by the use of the inventive combinations or compositions, which are applied to the seed, the plant or plant parts, the fruit or the soil in which the plants grow.

*Plants*

All plants and plant parts can be treated in accordance with the invention. Plants are understood here to mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the transgenic plants and including the plant cultivars which are protectable and non-protectable by plant breeders' rights. Plant parts are understood to mean all parts and organs of plants above and below the ground, such as shoot, leaf, flower and root, examples of which include leaves, needles, stalks, stems, flowers, fruit bodies, fruits and seeds, and also roots, tubers and rhizomes. The plant parts also include harvested material and vegetative and generative propagation material, for example cuttings, tubers, rhizomes, slips and seeds.

Plants which can be treated in accordance with the invention include the following: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

*Pathogens*

Non-limiting examples of pathogens of fungal diseases which can be treated in accordance with the invention include:

diseases caused by powdery mildew pathogens, for example Blumeria species, for example Blumeria graminis; Podosphaera species, for example Podosphaera leucotricha; Sphaerotheca species, for example Sphaerotheca fuliginea; Uncinula species, for example Uncinula necator;

diseases caused by rust disease pathogens, for example Gymnosporangium species, for example Gymnosporangium sabinae; Hemileia species, for example Hemileia vastatrix; Phakopsora species, for example Phakopsora pachyrhizi or Phakopsora meibomiae; Puccinia species, for example Puccinia recondita, Puccinia graminis oder Puccinia striiformis; Uromyces species, for example Uromyces appendiculatus;

diseases caused by pathogens from the group of the Oomycetes, for example Albugo species, for example Albugo candida; Bremia species, for example Bremia lactucae; Peronospora species, for example Peronospora pisi or P. brassicae; Phytophthora species, for example Phytophthora infestans; Plasmopara species, for example Plasmopara viticola; Pseudoperonospora species, for example Pseudoperonospora humuli or Pseudoperonospora cubensis; Pythium species, for example Pythium ultimum;

leaf blotch diseases and leaf wilt diseases caused, for example, by Alternaria species, for example Alternaria solani; Cercospora species, for example Cercospora beticola; Cladiosporium species, for example Cladiosporium cucumerinum; Cochliobolus species, for example Cochliobolus sativus (conidial form: Drechslera, syn: Helminthosporium) or Cochliobolus miyabeanus; Colletotrichum species, for example Colletotrichum lindemuthanium; Cycloconium species, for example Cycloconium oleaginum; Diaporthe species, for example Diaporthe citri; Elsinoe species, for example Elsinoe fawcettii; Gloeosporium species, for example Gloeosporium laeticolor; Glomerella species, for example Glomerella cingulata; Guignardia species, for example Guignardia bidwelli; Leptosphaeria species, for example Leptosphaeria maculans; Magnaporthe species, for example Magnaporthe grisea; Microdochium species, for example Microdochium nivale; Mycosphaerella species, for example Mycosphaerella graminicola, Mycosphaerella arachidicola or Mycosphaerella fijiensis; Phaeosphaeria species, for example Phaeosphaeria nodorum; Pyrenophora species, for example Pyrenophora teres or Pyrenophora tritici repentis; Ramularia species, for example Ramularia collo-cygni or Ramularia areola; Rhynchosporium species, for example Rhynchosporium secalis; Septoria species, for example Septoria apii or Septoria lycopersici; Stagonospora species, for example Stagonospora nodorum; Typhula species, for example Typhula incarnata; Venturia species, for example Venturia inaequalis;

root and stem diseases caused, for example, by Corticium species, for example Corticium graminearum; Fusarium species, for example Fusarium oxysporum; Gaeumannomyces species, for example Gaeumannomyces graminis; Plasmodiophora species, for example Plasmodiophora brassicae; Rhizoctonia species, for example Rhizoctonia solani; Sarocladium species, for example Sarocladium oryzae; Sclerotium species, for example Sclerotium oryzae; Tapesia species, for example Tapesia acuformis; Thielaviopsis species, for example Thielaviopsis basicola;

ear and panicle diseases (including corn cobs) caused, for example, by Alternaria species, for example Alternaria spp.; Aspergillus species, for example Aspergillus flavus; Cladosporium species, for example Cladosporium cladosporioides; Claviceps species, for example Claviceps purpurea; Fusarium species, for example Fusarium culmorum; Gibberella species, for example Gibberella zeae; Monographella species, for example Monographella nivalis; Stagnospora species, for example Stagnospora nodorum;

diseases caused by smut fungi, for example Sphacelotheca species, for example Sphacelotheca reiliana; Tilletia species, for example Tilletia caries or Tilletia controversa; Urocystis species, for example Urocystis occulta; Ustilago species, for example Ustilago nuda;

fruit rot caused, for example, by Aspergillus species, for example Aspergillus flavus; Botrytis species, for example Botrytis cinerea; Penicillium species, for example Penicillium expansum or Penicillium purpurogenum; Rhizopus species, for example Rhizopus stolonifer; Sclerotinia species, for example Sclerotinia sclerotiorum; Verticilium species, for example Verticilium alboatrum;

seed- and soil-borne rot and wilt diseases, and also diseases of seedlings, caused, for example, by Alternaria species, for example Alternaria brassicicola; Aphanomyces species, for example Aphanomyces euteiches; Ascochyta species, for example Ascochyta lentis; Aspergillus species, for example Aspergillus flavus; Cladosporium species, for example Cladosporium herbarum; Cochliobolus species, for example Cochliobolus sativus (conidial form: Drechslera, Bipolaris Syn: Helminthosporium); Colletotrichum species, for example Colletotrichum coccodes; Fusarium species, for example Fusarium culmorum; Gibberella species, for example Gibberella zeae; Macrophomina species, for example Macrophomina phaseolina; Microdochium species, for example Microdochium nivale; Monographella species, for example Monographella nivalis; Penicillium species, for example Penicillium expansum; Phoma species, for example Phoma lingam; Phomopsis species, for example Phomopsis sojae; Phytophthora species, for example Phytophthora cactorum; Pyrenophora species, for example Pyrenophora graminea; Pyricularia species, for example Pyricularia oryzae; Pythium species, for example Pythium ultimum; Rhizoctonia species, for example Rhizoctonia solani; Rhizopus species, for example Rhizopus oryzae; Sclerotium species, for example Sclerotium rolfsii; Septoria species, for example Septoria nodorum; Typhula species, for example Typhula incarnata; Verticillium species, for example Verticillium dahliae;

cancers, galls and witches' broom caused, for example, by Nectria species, for example Nectria galligena;

wilt diseases caused, for example, by Monilinia species, for example Monilinia laxa;

deformations of leaves, flowers and fruits caused, for example, by Exobasidium species, for example Exobasidium vexans; Taphrina species, for example Taphrina deformans;

degenerative diseases in woody plants, caused, for example, by Esca species, for example Phaeomoniella chlamydospora, Phaeoacremonium aleophilum or Fomitiporia mediterranea; Ganoderma species, for example Ganoderma boninense;

diseases of flowers and seeds caused, for example, by Botrytis species, for example Botrytis cinerea;

diseases of plant tubers caused, for example, by Rhizoctonia species, for example Rhizoctonia solani; Helminthosporium species, for example Helminthosporium solani;

diseases caused by bacterial pathogens, for example Xanthomonas species, for example Xanthomonas campestris pv. oryzae; Pseudomonas species, for example Pseudomonas syringae pv. lachrymans; Erwinia species, for example Erwinia amylovora.

Preference is given to controlling the following diseases of soya beans:
Fungal diseases on leaves, stems, pods and seeds caused, for example, by Alternaria leaf spot (Alternaria spec. atrans tenuissima), Anthracnose (Colletotrichum gloeosporoides dematium var. truncatum), brown spot (Septoria glycines), cercospora leaf spot and blight (Cercospora kikuchii), choanephora leaf blight (Choanephora infundibulifera trispora (Syn.)), dactuliophora leaf spot (Dactuliophora glycines), downy mildew (Peronospora manshurica), drechslera blight (Drechslera glycini), frogeye leaf spot (Cercospora sojina), leptosphaerulina leaf spot (Leptosphaerulina trifolii), phyllostica leaf spot (Phyllosticta sojaecola), pod and stem blight (Phomopsis sojae), powdery mildew (Microsphaera diffusa), pyrenochaeta leaf spot (Pyrenochaeta glycines), rhizoctonia aerial, foliage, and web blight (Rhizoctonia solani), rust (Phakopsora pachyrhizi, Phakopsora meibomiae), scab (Sphaceloma glycines), stemphylium leaf blight (Stemphylium botryosum), target spot (Corynespora cassiicola).

Fungal diseases on roots and the stem base caused, for example, by black root rot (Calonectria crotalariae), charcoal rot (Macrophomina phaseolina), fusarium blight or wilt, root rot, and pod and collar rot (Fusarium oxysporum, Fusarium orthoceras, Fusarium semitectum, Fusarium equiseti), mycoleptodiscus root rot (Mycoleptodiscus terrestris), neocosmospora (Neocosmospora vasinfecta), pod and stem blight (Diaporthe phaseolorum), stem canker (Diaporthe phaseolorum var. caulivora), phytophthora rot (Phytophthora megasperma), brown stem rot (Phialophora gregata), pythium rot (Pythium aphanidermatum, Pythium irregulare, Pythium debaryanum, Pythium myriotylum, Pythium ultimum), rhizoctonia root rot, stem decay, and damping-off (Rhizoctonia solani), sclerotinia stem decay (Sclerotinia sclerotiorum), sclerotinia southern blight (Sclerotinia rolfsii), thielaviopsis root rot (Thielaviopsis basicola).

Preference is further given to controlling leaf blotch diseases and leaf wilt diseases as well as root and stem diseases of fruits and vegetables.

*Plant Growth Regulation*

In some cases, the compound combinations according to the invention and composition comprising such combination can, at particular concentrations or application rates, also be used as growth regulators or agents to improve plant properties, or as microbicides, for example as fungicides, antimycotics, bactericides, viricides (including compositions against viroids) or as compositions against MLO (Mycoplasma-like organisms) and RLO (Rickettsia-like organisms).

The compound combinations according to the invention and compositions comprising such combination intervene in physiological processes of plants and can therefore also be used as plant growth regulators. Plant growth regulators may exert various effects on plants. The effect of the substances depends essentially on the time of application in relation to the developmental stage of the plant, and also on the amounts of active ingredient applied to the plants or their environment and on the type of application. In each case, growth regulators should have a particular desired effect on the crop plants.

Growth regulating effects, comprise earlier germination, better emergence, more developed root system and/or improved root growth, increased ability of tillering, more productive tillers, earlier flowering, increased plant height and/or biomass, shorting of stems, improvements in shoot growth, number of kernels/ear, number of ears/$m^2$, number of stolons and/or number of flowers, enhanced harvest index, bigger leaves, less dead basal leaves, improved phyllotaxy, earlier maturation / earlier fruit finish, homogenous riping, increased duration of grain filling, better fruit finish, bigger fruit/vegetable size, sprouting resistance and reduced lodging.

Increased or improved yield is referring to total biomass per hectare, yield per hectare, kernel/fruit weight, seed size and/or hectolitre weight as well as to improved product quality, comprising:

improved processability relating to size distribution (kernel, fruit, etc.), homogenous riping, grain moisture, better milling, better vinification, better brewing, increased juice yield, harvestability, digestibility, sedimentation value,

falling number, pod stability, storage stability, improved fiber length/strength/uniformity, increase of milk and/or meet quality of silage fed animals, adaption to cooking and frying;

further comprising improved marketability relating to improved fruit/grain quality, size distribution (kernel, fruit, etc.), increased storage / shelf-life, firmness / softness, taste (aroma, texture, etc.), grade (size, shape, number of berries, etc.), number of berries/fruits per bunch, crispness, freshness, coverage with wax, frequency of physiological disorders, colour, etc.;

further comprising increased desired ingredients such as e.g. protein content, fatty acids, oil content, oil quality, aminoacid composition, sugar content, acid content (pH), sugar/acid ratio (Brix), polyphenols, starch content, nutritional quality, gluten content/index, energy content, taste, etc.;

and further comprising decreased undesired ingredients such as e.g. less mycotoxines, less aflatoxines, geosmin level, phenolic aromas, lacchase, polyphenol oxidases and peroxidases, nitrate content etc.

Plant growth-regulating compounds can be used, for example, to slow down the vegetative growth of the plants. Such growth depression is of economic interest, for example, in the case of grasses, since it is thus possible to reduce the frequency of grass cutting in ornamental gardens, parks and sport facilities, on roadsides, at airports or in fruit crops. Also of significance is the inhibition of the growth of herbaceous and woody plants on roadsides and in the vicinity of pipelines or overhead cables, or quite generally in areas where vigorous plant growth is unwanted.

Also important is the use of growth regulators for inhibition of the longitudinal growth of cereal. This reduces or completely eliminates the risk of lodging of the plants prior to harvest. In addition, growth regulators in the case of cereals can strengthen the culm, which also counteracts lodging. The employment of growth regulators for shortening and strengthening culms allows the deployment of higher fertilizer volumes to increase the yield, without any risk of lodging of the cereal crop.

In many crop plants, vegetative growth depression allows denser planting, and it is thus possible to achieve higher yields based on the soil surface. Another advantage of the smaller plants obtained in this way is that the crop is easier to cultivate and harvest.

Reduction of the vegetative plant growth may also lead to increased or improved yields because the nutrients and assimilates are of more benefit to flower and fruit formation than to the vegetative parts of the plants.

Alternatively, growth regulators can also be used to promote vegetative growth. This is of great benefit when harvesting the vegetative plant parts. However, promoting vegetative growth may also promote generative growth in that more assimilates are formed, resulting in more or larger fruits.

Furthermore, beneficial effects on growth or yield can be achieved through improved nutrient use efficiency, especially nitrogen (N)-use efficiency, phosphours (P)-use efficiency, water use efficiency, improved transpiration, respiration and/or $CO_2$ assimilation rate, better nodulation, improved Ca-metabolism etc.

Likewise, growth regulators can be used to alter the composition of the plants, which in turn may result in an improvement in quality of the harvested products. Under the influence of growth regulators, parthenocarpic fruits may be formed. In addition, it is possible to influence the sex of the flowers. It is also possible to produce sterile pollen, which is of great importance in the breeding and production of hybrid seed.

Use of growth regulators can control the branching of the plants. On the one hand, by breaking apical dominance, it is possible to promote the development of side shoots, which may be highly desirable particularly in the cultivation of ornamental plants, also in combination with an inhibition of growth. On the other hand, however, it is also possible to inhibit the growth of the side shoots. This effect is of particular interest, for example, in the cultivation of tobacco or in the cultivation of tomatoes.

Under the influence of growth regulators, the amount of leaves on the plants can be controlled such that defoliation of the plants is achieved at a desired time. Such defoliation plays a major role in the mechanical harvesting of cotton, but is also of interest for facilitating harvesting in other crops, for example in viticulture. Defoliation of the plants can also be undertaken to lower the transpiration of the plants before they are transplanted.

Furthermore, growth regulators can modulate plant senescence, which may result in prolonged green leaf area duration, a longer grain filling phase, improved yield quality, etc.

Growth regulators can likewise be used to regulate fruit dehiscence. On the one hand, it is possible to prevent premature fruit dehiscence. On the other hand, it is also possible to promote fruit dehiscence or even flower abortion to achieve a desired mass ("thinning"). In addition it is possible to use growth regulators at the time of harvest to reduce the forces required to detach the fruits, in order to allow mechanical harvesting or to facilitate manual harvesting.

Growth regulators can also be used to achieve faster or else delayed ripening of the harvested material before or after harvest. This is particularly advantageous as it allows optimal adjustment to the requirements of the market. Moreover, growth regulators in some cases can improve the fruit colour. In addition, growth regulators can also be used

to synchronize maturation within a certain period of time. This establishes the prerequisites for complete mechanical or manual harvesting in a single operation, for example in the case of tobacco, tomatoes or coffee.

By using growth regulators, it is additionally possible to influence the resting of seed or buds of the plants, such that plants such as pineapple or ornamental plants in nurseries, for example, germinate, sprout or flower at a time when they are normally not inclined to do so. In areas where there is a risk of frost, it may be desirable to delay budding or germination of seeds with the aid of growth regulators, in order to avoid damage resulting from late frosts.

Finally, growth regulators can induce resistance of the plants to frost, drought or high salinity of the soil. This allows the cultivation of plants in regions which are normally unsuitable for this purpose.

*Resistance Induction / Plant Health and other effects*

The compound combinations according to the invention and compositions comprising such combination may also exhibit a potent strengthening effect in plants. Accordingly, they can be used for mobilizing the defences of the plant against attack by undesirable microorganisms.

Plant-strengthening (resistance-inducing) substances in the present context are substances capable of stimulating the defence system of plants in such a way that the treated plants, when subsequently inoculated with undesirable microorganisms, develop a high degree of resistance to these microorganisms.

Further, in context with the present invention plant physiology effects comprise the following:

Abiotic stress tolerance, comprising tolerance to high or low temperatures, drought tolerance and recovery after drought stress, water use efficiency (correlating to reduced water consumption), flood tolerance, ozone stress and UV tolerance, tolerance towards chemicals like heavy metals, salts, pesticides etc.

Biotic stress tolerance, comprising increased fungal resistance and increased resistance against nematodes, viruses and bacteria. In context with the present invention, biotic stress tolerance preferably comprises increased fungal resistance and increased resistance against nematodes.

Increased plant vigor, comprising plant health / plant quality and seed vigor, reduced stand failure, improved appearance, increased recovery after periods of stress, improved pigmentation (e.g. chlorophyll content, stay-green effects, etc.) and improved photosynthetic efficiency.

*Mycotoxins*

In addition, the compound combinations according to the invention and compositions comprising such combination can reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides* etc., and also by *Aspergillus* spec., such as *A. flavus, A. parasiticus, A. nomius, A. ochraceus, A. clavatus, A. terreus, A. versicolor, Penicillium* spec., such as *P. verrucosum, P. viridicatum, P. citrinum, P. expansum, P. claviforme, P. roqueforti, Claviceps* spec., such as C. *purpurea, C. fusiformis, C. paspali, C. africana, Stachybotrys* spec. and others.

*Material Protection*

The compound combinations according to the invention and compositions comprising such combination can also be used in the protection of materials, for protection of industrial materials against attack and destruction by phytopathogenic fungi.

In addition, the compound combinations according to the invention and compositions comprising such combination can be used as antifouling compositions, alone or in combinations with other active ingredients.

Industrial materials in the present context are understood to mean inanimate materials which have been prepared for use in industry. For example, industrial materials which are to be protected by inventive compositions from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants

and heat transfer fluids, more preferably wood.

The compound combinations according to the invention and compositions comprising such combination may prevent adverse effects, such as rotting, decay, discoloration, decoloration or formation of mould.

In the case of treatment of wood the compound combinations according to the invention and compositions comprising such combination may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting a composition according to the invention; this includes for example direct application, spraying, dipping, injection or any other suitable means.

In addition, the compound combinations according to the invention and compositions comprising such combination can be used to protect objects which come into contact with saltwater or brackish water, especially hulls, screens, nets, buildings, moorings and signalling systems, from fouling.

The compound combinations according to the invention and compositions comprising such combination can also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, can be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The inventive compositions may prevent adverse effects, such as rotting, decay, discoloration, decoloration or formation of mould.

Microorganisms capable of degrading or altering the industrial materials include, for example, bacteria, fungi, yeasts, algae and slime organisms. The compounds of the formula (I) preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis; Aspergillus,* such as *Aspergillus niger; Chaetomium,* such as *Chaetomium globosum; Coniophora,* such as *Coniophora puetana; Lentinus,* such as *Lentinus tigrinus; Penicillium,* such as *Penicillium glaucum; Polyporus,* such as *Polyporus versicolor; Aureobasidium,* such as *Aureobasidium pullulans; Sclerophoma,* such as *Sclerophoma pityophila; Trichoderma,* such as *Trichoderma viride; Ophiostoma spp., Ceratocystis spp., Humicola spp., Petriella spp., Trichurus spp., Coriolus spp., Gloeophyllum spp., Pleurotus spp., Poria spp., Serpula spp.* and *Tyromyces spp., Cladosporium spp., Paecilomyces spp. Mucor spp., Escherichia,* such as *Escherichia coli; Pseudomonas,* such as *Pseudomonas aeruginosa; Staphylococcus,* such as *Staphylococcus aureus, Candida spp.* and *Saccharomyces spp.,* such as *Saccharomyces cerevisae.*

*Formulations*

The present invention further relates to a composition for controlling unwanted microorganisms, comprising compound combinations according to the invention. These are preferably fungicidal compositions which comprise agriculturally suitable auxiliaries, like solvents, carriers, surfactants or extenders.

According to the invention, a carrier is a natural or synthetic, organic or inorganic substance with which the active ingredients are mixed or combined for better applicability, in particular for application to plants or plant parts or seed. The carrier, which may be solid or liquid, is generally inert and should be suitable for use in agriculture.

Useful solid carriers include: for example ammonium salts and natural rock flours, such as kaolins, clays, talc, chalk, quartz, attapulgite, montmorillonite or diatomaceous earth, and synthetic rock flours, such as finely divided silica, alumina and silicates; useful solid carriers for granules include: for example, crushed and fractionated natural rocks such as calcite, marble, pumice, sepiolite and dolomite, and also synthetic granules of inorganic and organic flours, and granules of organic material such as paper, sawdust, coconut shells, maize cobs and tobacco stalks.

Useful emulsifiers and/or foam-formers include: for example nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates.

Suitable dispersants are nonionic and/or ionic substances, for example from the classes of the alcohol-POE and/or -POP ethers, acid and/or POP POE esters, alkylaryl and/or POP POE ethers, fat and/or POP POE adducts, POE- and/or POP-polyol derivatives, POE- and/or POP-sorbitan or -sugar adducts, alkyl or aryl sulphates, alkyl- or arylsulphonates and alkyl or aryl phosphates or the corresponding PO-ether adducts. Additionally suitable are oligo- or polymers, for example those derived from vinylic monomers, from acrylic acid, from EO and/or PO alone or in combination with, for example, (poly)alcohols or (poly)amines. It is also possible to use lignin and its sulphonic acid derivatives, unmodified and modified celluloses, aromatic and/or aliphatic sulphonic acids and also their adducts with formaldehyde.

The active ingredients can be converted to the customary formulations, such as solutions, emulsions, wettable

powders, water- and oil-based suspensions, powders, dusts, pastes, soluble powders, soluble granules, granules for broadcasting, suspoemulsion concentrates, natural products impregnated with active ingredient, synthetic substances impregnated with active ingredient, fertilizers and also microencapsulations in polymeric substances.

The active ingredients can be applied as such, in the form of their formulations or the use forms prepared therefrom, such as ready-to-use solutions, emulsions, water- or oil-based suspensions, powders, wettable powders, pastes, soluble powders, dusts, soluble granules, granules for broadcasting, suspoemulsion concentrates, natural products impregnated with active ingredient, synthetic substances impregnated with active ingredient, fertilizers and also microencapsulations in polymeric substances. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

The formulations mentioned can be prepared in a manner known per se, for example by mixing the active ingredients with at least one customary extender, solvent or diluent, emulsifier, dispersant and/or binder or fixing agent, wetting agent, a water repellent, if appropriate siccatives and UV stabilizers and if appropriate dyes and pigments, antifoams, preservatives, secondary thickeners, stickers, gibberellins and also other processing auxiliaries.

The present invention includes not only formulations which are already ready for use and can be deployed with a suitable apparatus to the plant or the seed, but also commercial concentrates which have to be diluted with water prior to use.

The compound combinations according to the invention may be present as such or in their (commercial) formulations and in the use forms prepared from these formulations as a mixture with other (known) active ingredients, such as insecticides, attractants, sterilants, bactericides, acaricides, nematicides, growth regulators, herbicides, fertilizers, safeners and/or semiochemicals.

The auxiliaries used may be those substances which are suitable for imparting particular properties to the composition itself or and/or to preparations derived therefrom (for example spray liquors, seed dressings), such as certain technical properties and/or also particular biological properties. Typical auxiliaries include: extenders, solvents and carriers.

Suitable extenders are, for example, water, polar and nonpolar organic chemical liquids, for example from the classes of the aromatic and nonaromatic hydrocarbons (such as paraffins, alkylbenzenes, alkylnaphthalenes, chlorobenzenes), the alcohols and polyols (which may optionally also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

Liquefied gaseous extenders or carriers are understood to mean liquids which are gaseous at standard temperature and under standard pressure, for example aerosol propellants such as halohydrocarbons, or else butane, propane, nitrogen and carbon dioxide.

In the formulations it is possible to use tackifiers such as carboxymethylcellulose, natural and synthetic polymers in the form of powders, granules or latices, such as gum arabic, polyvinyl alcohol and polyvinyl acetate, or else natural phospholipids such as cephalins and lecithins and synthetic phospholipids. Further additives may be mineral and vegetable oils.

If the extender used is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example petroleum fractions, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, or else water.

Compositions comprising compound combinations according to the invention may additionally comprise further components, for example surfactants. Suitable surfactants are emulsifiers and/or foam formers, dispersants or wetting agents having ionic or nonionic properties, or mixtures of these surfactants. Examples thereof are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. The presence of a surfactant is necessary if one of the active ingredients and/or one of the inert carriers is insoluble in water and when application is effected in water. The proportion of surfactants is between 5 and 40 per cent by weight of the inventive composition.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of

iron, manganese, boron, copper, cobalt, molybdenum and zinc.

Further additives may be perfumes, mineral or vegetable, optionally modified oils, waxes and nutrients (including trace nutrients), such as salts of iron, manganese, boron, copper, cobalt, molybdenum and zinc.

Additional components may be stabilizers, such as cold stabilizers, preservatives, antioxidants, light stabilizers, or other agents which improve chemical and/or physical stability.

If appropriate, other additional components may also be present, for example protective colloids, binders, adhesives, thickeners, thixotropic substances, penetrants, stabilizers, sequestering agents, complex formers. In general, the active ingredients can be combined with any solid or liquid additive commonly used for formulation purposes.

The formulations contain generally between 0.05 and 99% by weight, 0.01 and 98% by weight, preferably between 0.1 and 95% by weight, more preferably between 0.5 and 90% of active ingredient, most preferably between 10 and 70 per cent by weight.

The formulations described above can be used for controlling unwanted microorganisms by applying it to the microorganisms and/or in their habitat.

In one particular embodiment of the invention, the compound combination is provided in sprayable form to allow spray application. In this embodiment, the compound combination according to the invention is provided as composition / formulation, comprising the active ingredients and at least one suitable liquid carrier.

Suitable liquid carriers are preferably selected from water, organic solvents and combinations thereof. More preferred, the liquid carrier is water or a mixture of water and an organic solvent.

Examples of suitable organic solvents have already described above.

The amount of liquid carrier typically ranges from 1 to 99.99%, preferably from 5 to 99.9%, more preferably from 10 to 99.5%, and most preferably from 20 to 99% by weight of the composition.

Preferably, the sprayable composition further comprises at least one surfactant. Suitable surfactants are disclosed above. It may also comprise at least one further auxiliary as outlined above.

Preferably, the sprayable composition is provided as an emulsifiable concentrate or suspension concentrate, more preferably an emulsifiable concentrate comprising the active ingredients in a total amount of 20 to 400 g/L, preferably 40 to 200 g/L, or a suspension concentrate comprising the active ingredients in a total amount of 50 to 500 g/L, preferably 100 to 400 g/L.

Preparation of said concentrates is well known to those skilled in the art. For example, emulsifiable concentrates (EC) can be prepared by dissolving the desired amount of active ingredients, e.g. 20 to 400 g per litre concentrate, and 50 to 100 g per litre concentrate of at least one surfactant in a water-insoluble organic solvent, e.g. an aromatic hydrocarbon, or a water-soluble organic solvent, e.g. N-methyl-2-pyrrolidone (NMP), dimethylsulfoxide (DMSO) and γ-butyrolactone. Suspension concentrates (SC) can be prepared by mixing the desired amount of active ingredients, e.g. 50 to 500 g per litre concentrate, 20 to 100 g per litre concentrate of at least one surfactant and 1 to 20 g per litre concentrate of at least one binder and/or secondary thickener and suspending this mixture in water.

Preferably, before applying said concentrates to the plant or a part thereof, the concentrate is diluted with water. More preferably, the emulsifiable concentrate or suspension concentrate is mixed with water in such amount to arrive at a total concentration of active ingredients in the resulting mixture of 0.1 to 5, preferably 0.2 to 2, more preferred 0.25 to 1 g/l.

*Mixtures*

Compound combinations according to the invention can be used as such or in formulations thereof and can be mixed with known bactericides, acaricides, nematicides or insecticides, in order thus to broaden, for example, the activity spectrum or to prevent development of resistance.

Useful mixing partners include, for example, known insecticides, acaricides, nematicides or else bactericides (see also Pesticide Manual, 14th ed.).

A mixture with other known active ingredients, such as herbicides, or with fertilizers and growth regulators, safeners and/or semiochemicals, is also possible.

*Seed Treatment*

The invention furthermore includes a method for treating seed.

A further aspect of the present invention relates in particular to seeds (dormant, primed, pregerminated or even with emerged roots and leaves) treated with a compound combination according to the invention or a composition comprising such combination. The inventive seeds are used in methods for protection of seeds and emerged plants from the seeds from phytopathogenic harmful fungi. In these methods, seed treated with at least one inventive active ingredient is used.

The compound combinations according to the invention and compositions comprising such combination are

also suitable for the treatment of seeds and young seedlings. A large part of the damage to crop plants caused by harmful organisms is triggered by the infection of the seeds before sowing or after germination of the plant. This phase is particularly critical since the roots and shoots of the growing plant are particularly sensitive, and even small damage may result in the death of the plant. Accordingly, there is great interest in protecting the seed and the germinating plant by using appropriate compositions.

It is also desirable to optimize the amount of the active ingredient used so as to provide the best possible protection for the seeds, the germinating plants and emerged seedlings from attack by phytopathogenic fungi, but without damaging the plants themselves by the active ingredient used. In particular, methods for the treatment of seed should also take into consideration the intrinsic phenotypes of transgenic plants in order to achieve optimum protection of the seed and the germinating plant with a minimum of crop protection compositions being employed.

The present invention therefore also relates to a method for protecting seeds, germinating plants and emerged seedlings against attack by animal pests and/or phytopathogenic harmful microorganisms by treating the seeds with an inventive combination or composition. The invention also relates to the use of the combinations or compositions according to the invention for treating seeds for protecting the seeds, the germinating plants and emerged seedlings against animal pests and/or phytopathogenic microorganisms. The invention further relates to seeds which have been treated with an inventive combination or composition for protection from animal pests and/or phytopathogenic microorganisms.

One of the advantages of the present invention is that the treatment of the seeds with these compositions not only protects the seed itself, but also the resulting plants after emergence, from animal pests and/or phytopathogenic harmful microorganisms. In this way, the immediate treatment of the crop at the time of sowing or shortly thereafter protect plants as well as seed treatment in prior to sowing. It is likewise considered to be advantageous that the inventive active ingredients combination or composition can be used especially also for transgenic seed, in which case the plant which grows from this seed is capable of expressing a protein which acts against pests, herbicidal damage or abiotic stress. The treatment of such seeds with the inventive active ingredients or compositions, for example an insecticidal protein, can result in control of certain pests.

The compound combinations according to the invention and compositions comprising such combination are suitable for protection of seed of any plant variety which is used in agriculture, in the greenhouse, in forests or in horticulture. More particularly, the seed is that of cereals (such as wheat, barley, rye, millet and oats), oilseed rape, maize, cotton, soybeen, rice, potatoes, sunflower, beans, coffee, beet (e.g. sugar beet and fodder beet), peanut, vegetables (such as tomato, cucumber, onions and lettuce), lawns and ornamental plants. Of particular significance is the treatment of the seed of wheat, soybean, oilseed rape, maize and rice.

As also described below, the treatment of transgenic seed with the inventive active ingredients or compositions is of particular significance. This refers to the seed of plants containing at least one heterologous gene which allows the expression of a polypeptide or protein, e.g. having insecticidal properties. These heterologous genes in transgenic seeds may originate, for example, from microorganisms of the species Bacillus, Rhizobium, Pseudomonas, Serratia, Trichoderma, Clavibacter, Glomus or Gliocladium. These heterologous genes preferably originate from Bacillus sp., in which case the gene product is effective against the European corn borer and/or the Western corn rootworm. Particularly preferably, the heterologous genes originate from Bacillus thuringiensis.

In the context of the present invention, the inventive combination or composition is applied to seeds either alone or in a suitable formulation. Preferably, the seed is treated in a state in which it is sufficiently stable for no damage to occur in the course of treatment. In general, seeds can be treated at any time between harvest and some time after sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15% by weight. Alternatively, it is also possible to use seed which, after drying, for example, has been treated with water and then dried again, or seeds just after priming, or seeds stored in primed conditions or pre-germinated seeds, or seeds sown on nursery trays, tapes or paper.

When treating the seeds, it generally has to be ensured that the amount of the inventive combination or composition applied to the seed and/or the amount of further additives is selected such that the germination of the seed is not impaired, or that the resulting plant is not damaged. This must be ensured particularly in the case of active ingredients which can exhibit phytotoxic effects at certain application rates.

The compound combinations according to the invention and compositions comprising such combination can be applied directly, i.e. without containing any other components and without having been diluted. In general, it is preferable to apply the compositions to the seed in the form of a suitable formulation. Suitable formulations and methods for seed treatment are known to those skilled in the art. The compound combinations according to the invention can be converted to the customary formulations relevant to on-seed applications, such as solutions, emulsions, suspensions, powders, foams, slurries or combined with other coating compositions for seed, such as film forming materials, pelleting materials, fine iron or other metal powders, granules, coating material for inactivated seeds, and also ULV formulations.

These formulations are prepared in a known manner, by mixing the active ingredients or active ingredient combinations with customary additives, for example customary extenders and solvents or diluents, dyes, wetting agents,

dispersants, emulsifiers, antifoams, preservatives, secondary thickeners, adhesives, gibberellins, and also water.

Useful dyes which may be present in the seed dressing formulations usable in accordance with the invention are all dyes which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of active agrochemical ingredients. Usable with preference are alkylnaphthalenesulphonates, such as diisopropyl- or diisobutylnaphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Useful nonionic dispersants include especially ethylene oxide/propylene oxide block polymers, alkylphenol polyglycol ethers and tristryrylphenol polyglycol ether, and the phosphated or sulphated derivatives thereof. Suitable anionic dispersants are especially lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Silicone antifoams and magnesium stearate can be used with preference.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances usable for such purposes in agrochemical compositions. Preferred examples include cellulose derivatives, acrylic acid derivatives, xanthan, modified clays and finely divided silica.

Adhesives which may be present in the seed dressing formulations usable in accordance with the invention are all customary binders usable in seed dressing products. Preferred examples include polyvinylpyrrolidone, polyvinyl acetate, polyvinyl alcohol and tylose.

The formulations for on-seed applications usable in accordance with the invention can be used to treat a wide variety of different kinds of seed either directly or after prior dilution with water. For instance, the concentrates or the preparations obtainable therefrom by dilution with water can be used to dress the seed of cereals, such as wheat, barley, rye, oats, and triticale, and also seeds of maize, soybean, rice, oilseed rape, peas, beans, cotton, sunflowers, and beets, or else a wide variety of different vegetable seeds. The formulations usable in accordance with the invention, or the dilute preparations thereof, can also be used for seeds of transgenic plants. In this case, additional synergistic effects may also occur in interaction with the substances formed by expression.

For treatment of seeds with the formulations usable in accordance with the invention, or the preparations prepared therefrom by adding water, all mixing units usable customarily for on-seed applications are useful. Specifically, the procedure in on-seed applications is to place the seeds into a mixer, to add the particular desired amount of the formulations, either as such or after prior dilution with water, and to mix everything until all applied formulations are distributed homogeneously on the seeds. If appropriate, this is followed by a drying operation.

The application rate of the formulations usable in accordance with the invention can be varied within a relatively wide range. It is guided by the particular content of the active ingredients in the formulations and by the seeds. The application rate of each single active ingredient is generally between 0.001 and 15 g per kilogram of seed, preferably between 0.01 and 5 g per kilogram of seed.

*Antimycotic Effects*

In addition, the compound combinations according to the invention and compositions comprising such combination also have very good antimycotic effects. They have a very broad antimycotic activity spectrum, especially against dermatophytes and yeasts, moulds and diphasic fungi (for example against Candida species, such as Candida albicans, Candida glabrata), and Epidermophyton floccosum, Aspergillus species, such as Aspergillus niger and Aspergillus fumigatus, Trichophyton species, such as Trichophyton mentagrophytes, Microsporon species such as Microsporon canis and audouinii. The enumeration of these fungi by no means constitutes a restriction of the mycotic spectrum covered, and is merely of illustrative character.

The compound combinations according to the invention and compositions comprising such combination can be used also to control important fungal pathogens in fish and crustacea farming, e.g. saprolegnia diclina in trouts, saprolegnia parasitica in crayfish.

The compound combinations according to the invention and compositions comprising such combination can

be used as such, in the form of their formulations or the use forms prepared therefrom, such as ready-to-use solutions, suspensions, wettable powders, pastes, soluble powders, dusts and granules. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

*GMO*

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts" or "parts of plants" or "plant parts" have been explained above. More preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance.
Plants or plant cultivars (obtained by plant biotechnology methods such as genetic engineering) which may also be treated according to the invention show altered quantity, quality and/or storage-stability of the harvested product and/or altered properties of specific ingredients of the harvested product.
Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics.
Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics.
Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering.
Plants or plant cultivars (that can be obtained by plant biotechnology methods such as genetic engineering) which may also be treated according to the invention are plants, such as Tobacco plants, with altered post-translational protein modification patterns.

*Application Rates*

When using the compound combinations according to the invention and compositions comprising such combination as fungicides, the application rates can be varied within a relatively wide range, depending on the kind of application. The application rate is
in the case of treatment of plant parts, for example leaves: from 0.1 to 10 000 g/ha, preferably from 10 to 1000 g/ha, more preferably from 50 to 300 g/ha (in the case of application by watering or dripping, it is even possible to reduce the application rate, especially when inert substrates such as rockwool or perlite are used);
in the case of seed treatment: from 0.1 to 200 g per 100 kg of seed, preferably from 1 to 150 g per 100 kg of seed, more preferably from 2.5 to 25 g per 100 kg of seed, even more preferably from 2.5 to 12.5 g per 100 kg of seed;
in the case of soil treatment: from 0.1 to 10 000 g/ha, preferably from 1 to 5000 g/ha,
wherein the given amounts refer to the total amount of active ingredient in the respective combination or composition.
These application rates are merely by way of example and are not limiting for the purposes of the invention.
The invention is illustrated by the examples below. However, the invention is not limited to the examples.

## Examples

The advanced fungicidal activity of the active compound combinations according to the invention is evident from the examples below. While the individual active compounds exhibit weaknesses with regard to the fungicidal activity, the combinations have an activity which exceeds a simple addition of activities.
A synergistic effect of fungicides is always present when the fungicidal activity of the active compound combinations exceeds the total of the activities of the active compounds when applied individually. The expected activity for a given combination of two active compounds can be calculated as follows (cf. Colby, S.R., "Calculating Synergistic and Antagonistic Responses of Herbicide Combinations", Weeds 1967, 15, 20-22):
If

X is the efficacy when active compound A is applied at an application rate of m ppm (or g/ha),

Y is the efficacy when active compound B is applied at an application rate of n ppm (or g/ha),

E is the efficacy when the active compounds A and B are applied at application rates of m and n ppm (or g/ha), respectively, and

then

$$E = X + Y - \frac{X \cdot Y}{100}$$

The degree of efficacy, expressed in % is denoted. 0 % means an efficacy which corresponds to that of the control while an efficacy of 100 % means that no disease is observed.

If the actual fungicidal activity exceeds the calculated value, then the activity of the combination is superadditive, i.e. a synergistic effect exists. In this case, the efficacy which was actually observed must be greater than the value for the expected efficacy (E) calculated from the abovementioned formula.

A further way of demonstrating a synergistic effect is the method of Tammes (cf. "Isoboles, a graphic representation of synergism in pesticides" in Neth. J. Plant Path., 1964, 70, 73-80).

The invention is illustrated by the following examples. However the invention is not limited to the examples.

**Example A: *in vitro-Test* with fungal microorganisms**

Wells of 96-well microtiter plates are filled with 10μl of a preparation of test compound or compound combination in methanol + emulsifier alkylaryl-polyglycol-ether. Thereafter, the solvent is evaporated in a hood. At the next step, into each well 100μl of liquid potato dextrose medium is given, that has been amended with an appropriate concentration of spores or mycelium suspension of the test fungus.

With the aid of a photometer the extinction in all wells is measured at the wavelength of 620nm.

The microtiter plates are incubated at 20°C and 85% relative humidity. The inhibition of growth is determined again photometrically 3-5 days after the application. Efficacy is calculated in relation to the untreated control, 0% efficacy means fungal growth as high as in untreated control while 100% efficacy means no fungal growth is measured.

## Claims

1. Active compound combination comprising

(A) at least one triazole derivative of formula (I)

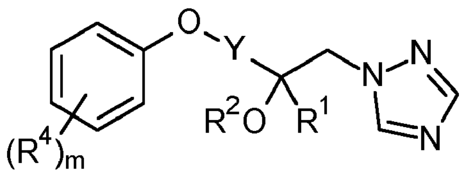

(I),

wherein

$R^1$ represents hydrogen, $C_1$-$C_6$-alkyl, $C_2$-$C_6$-alkenyl, $C_2$-$C_6$-alkynyl, $C_3$-$C_8$-cycloalkyl, $C_3$-$C_8$-cycloalkyl-$C_1$-$C_4$-alkyl or phenyl;
$R^2$ represents hydrogen, $C_1$-$C_6$-alkyl, $C_2$-$C_6$-alkenyl, $C_2$-$C_6$-alkynyl, $C_3$-$C_8$-cycloalkyl, $C_3$-$C_8$-cycloalkyl-$C_1$-$C_4$-alkyl or phenyl,
wherein the aliphatic moieties, excluding cycloalkyl moieties, of $R^1$ and $R^2$ may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-haloalkoxy, wherein the phenyl may be substituted by 1, 2, 3, 4 or 5 substituents selected from halogen, CN, nitro, $C_1$-$C_4$-alkyl, $C_1$-$C_4$-alkoxy, $C_1$-$C_4$-haloalkyl, $C_1$-$C_4$-haloalkoxy,
and wherein the cycloalkyl and/or phenyl moieties of $R^1$ and $R^2$ may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-haloalkyl and $C_1$-$C_4$-haloalkoxy;
each $R^4$ represents independently of one another halogen, CN, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylcarbonyl, hydroxy-substituted $C_1$-$C_4$-alkyl, pentafluoro-$\lambda^6$-sulfanyl, $C_3$-$C_6$-cycloalkyl, $C_3$-$C_6$-halocycloalkyl, $C_1$-$C_4$-alkyl-$C_3$-$C_6$-cycloalkyl, $C_2$-$C_6$-alkenyl, $C_2$-$C_6$-haloalkenyl, $C_2$-$C_6$-alkynyl, $C_2$-$C_6$-haloalkynyl, $C_1$-$C_4$-alkylsulfanyl, $C_1$-$C_4$-haloalkylsulfanyl, $C_1$-$C_6$-alkylsulfonyl, $C_6$-$C_{10}$-arylsulfonyl, $C_1$-$C_6$-alkyl-$SO_2NH$-, $C_6$-$C_{10}$-aryl-$SO_2NH$-, formyl, 5-, 6- or 7-membered saturated heterocycloalkyl containing up to 4 heteroatoms selected from N, O and S, or -C($R^{4a}$)=N-O$R^{4b}$, wherein $R^{4a}$ and $R^{4b}$ represent independently from each other hydrogen, $C_1$-$C_6$-alkyl or phenyl;
m is an integer and is 0, 1, 2, 3, 4 or 5;
Y represents a 6-membered aromatic cycle selected from

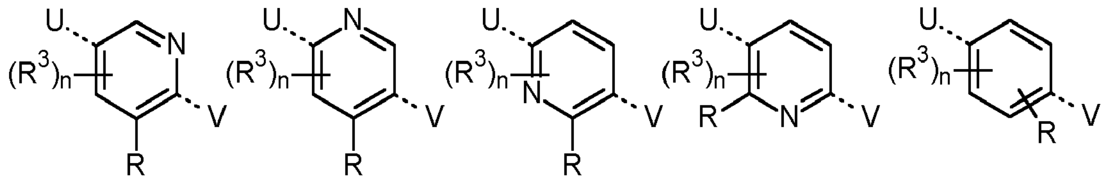

wherein Y is connected to the O of formula (I) via the bonds identified with "U" and Y connected to the $CR^1(OR^2)$ moiety of formula (I) via the bonds identified with "V" and wherein

R represents hydrogen, $C_1$-$C_2$-haloalkyl, $C_1$-$C_2$-haloalkoxy, $C_1$-$C_2$-alkylcarbonyl or halogen;
each $R^3$ represents independently of one another halogen, CN, nitro, $C_1$-$C_4$-alkyl, $C_1$-$C_4$-haloalkyl, $C_1$-$C_4$-alkoxy or $C_1$-$C_4$-haloalkoxy;
n is an integer and is 0, 1 or 2;

or a salt or N-oxide thereof, and
(B) florylpicoxamid or a salt or N-oxide thereof.

**2.** Active compound combination according to claim 1, wherein the triazole derivative of formula (I) is a triazole derivative of formula (I), wherein
each $R^4$ represents independently of one another halogen, CN, nitro, $C_1$-$C_4$-alkyl, $C_1$-$C_4$-haloalkyl, $C_1$-$C_4$-alkoxy, $C_1$-$C_4$-haloalkoxy or pentafluoro-$\lambda^6$-sulfanyl;
and/or
R represents hydrogen, $C_1$-$C_2$-haloalkyl or halogen,
or a salt or N-oxide thereof.

**3.** Active compound combination according to claim 1, wherein the triazole derivative of formula (I) is a triazole derivative of formula (I), wherein

Y represents a 6-membered aromatic heterocycle containing 1 or 2 nitrogen atom(s) as heteroatom(s) selected from

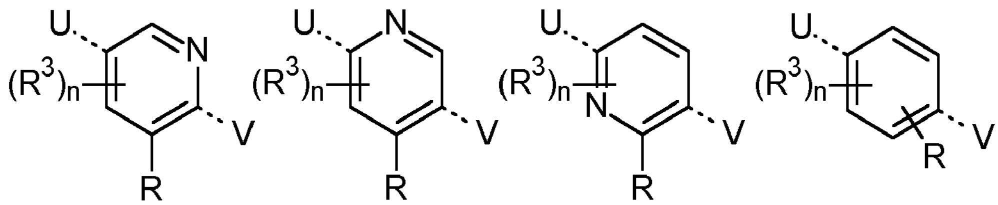

wherein Y is connected to the O of formula (I) via the bonds identified with "U" and Y is connected to the $CR^1(OR^2)$ moiety of formula (I) via the bonds identified with "V" and
wherein R, $R^3$ and n are defined according to claim 1,

or a salt or N-oxide thereof.

**4.** Active compound combination according to claim 1, wherein the triazole derivative of formula (I) is a triazole derivative of formula (I), wherein

$R^1$ represents hydrogen, $C_1$-$C_4$-alkyl, $C_2$-$C_6$-alkenyl, $C_2$-$C_6$-alkynyl, cyclopropyl or phenyl;
$R^2$ represents hydrogen, $C_1$-$C_4$-alkyl, allyl or prop-2-inyl,
wherein the aliphatic moieties, excluding cycloalkyl moieties, of $R^1$ and $R^2$ may carry 1, 2, 3 or up to the maximum possible number of identical or different groups $R^a$ which independently of one another are selected from

halogen, CN, nitro, phenyl, $C_1$-$C_4$-alkoxy and $C_1$-$C_4$-haloalkoxy, wherein the phenyl may be substituted by 1, 2, 3, 4 or 5 substituents selected independently of one another from halogen, CN, nitro, $C_1$-$C_4$-alkyl, $C_1$-$C_4$-alkoxy, $C_1$-$C_4$-haloalkyl and $C_1$-$C_4$-haloalkoxy,
and wherein the cycloalkyl and/or phenyl moieties of $R^1$ and $R^2$ may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-haloalkyl and $C_1$-$C_4$-haloalkoxy;
each $R^4$ represents independently of one another $CF_3$, $OCF_3$, Br, Cl or pentafluoro-$\lambda^6$-sulfanyl;
m is 1, 2 or 3;
Y represents

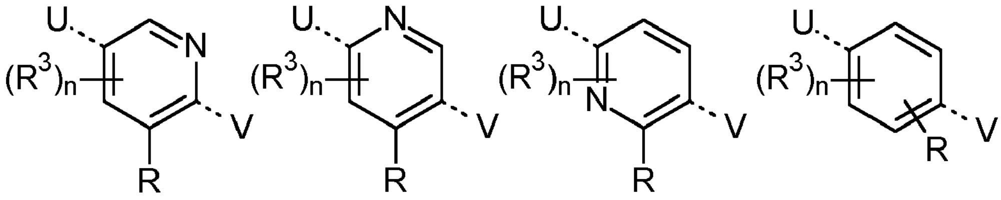

;

wherein Y is connected to the O of formula (I) via the bonds identified with "U" and Y is connected to the $CR^1(OR^2)$ moiety of formula (I) via the bonds identified with "V" and
wherein R, $R^3$ and n are defined according to claim 1;

or a salt or N-oxide thereof.

**5.** Active compound combination according to claim 1, wherein the triazole derivative of formula (I) is a triazole derivative of formula (I), wherein

$R^1$ represents hydrogen, $C_1$-$C_4$-alkyl or cyclopropyl;
$R^2$ represents hydrogen;
$R^4$ represents $CF_3$, $OCF_3$, Br, Cl or pentafluoro-$\lambda^6$-sulfanyl;
m is 1;
Y represents

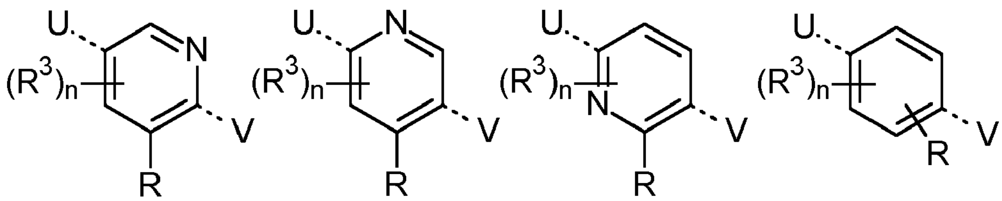

;

wherein Y is connected to the O of formula (I) via the bonds identified with "U" and Y is connected to the $CR^1(OR^2)$ moiety of formula (I) via the bonds identified with "V" and

R represents $C_1$-haloalkyl;
n is 0;

or a salt or N-oxide thereof.

**6.** Active compound combination according to at least one of claims 1 to 5, wherein the triazole derivative of formula (I) is a triazole derivative of formula (I), wherein
$R^4$ represents Cl or Br in the 4-position of the phenyl moiety of formula (I);
or a salt or N-oxide thereof.

**7.** Active compound combination according to at least one of claims 1 to 6, wherein the triazole derivative of formula (I) is selected from the group consisting of (I.01) 2-[6-(4-chlorophenoxy)-2-(trifluoromethyl)pyridin-3-yl]-1-(1H-1,2,4-triazol-1-yl)propan-2-ol, (I.59) 2-[6-(4-bromophenoxy)-2-(trifluoromethyl)pyridin-3-yl]-1-(1H-1,2,4-triazol-1-yl)propan-2-ol, (I.81) 1-[6-(4-bromophenoxy)-2-(trifluoromethyl)pyridin-3-yl]-1-cyclopropyl-2-(1H-1,2,4-triazol-1-yl)ethanol, (I.91) 1-[6-(4-chlorophenoxy)-2-(trifluoromethyl)pyridin-3-yl]-1-cyclopropyl-2-(1H-1,2,4-triazol-1-yl)ethanol, (I.104) 2-[4-(4-chlorophenoxy)-2-(trifluoromethyl)phenyl]-1-(1,2,4-triazol-1-yl)propan-2-ol and (I.105) 2-[4-(4-chlorophenoxy)-2-(trifluoromethyl)phenyl]-3-methyl-1-(1,2,4-triazol-1-yl)butan-2-ol.

**8.** Active compound combination according to at least one of claims 1 to 6, wherein the triazole derivative of formula (I) is selected from the group consisting of (I.01) 2-[6-(4-chlorophenoxy)-2-(trifluoromethyl)pyridin-3-yl]-1-(1H-1,2,4-triazol-1-yl)propan-2-ol, (I.59) 2-[6-(4-bromophenoxy)-2-(trifluoromethyl)pyridin-3 -yl] -1-(1H-1,2,4-triazol-1 -yl)propan-2-ol and (I.104) 2- [4-(4-chlorophenoxy)-2-(trifluoromethyl)phenyl] -1 -(1,2,4-triazol-1 -yl)propan-2-ol.

**9.** Active compound combination according to at least one of claims 1 to 8, comprising a further active compound (C) selected from the group consisting of (1.012) ipconazole, (1.018) prothioconazole, (1.020) spiroxamine, (1.021) tebuconazole, (2.002) bixafen, (2.005) fluopyram, (2.017) penflufen, (2.027) 3-(difluoromethyl)-1-methyl-N-(1,1,3-trimethyl-2,3-dihydro-1H-inden-4-yl)-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (3.020) trifloxystrobin, (3.025) (3S,6S,7R,8R)-8-benzyl-3-[({3-[(isobutyryloxy)methoxy]-4-methoxypyridin-2-yl}carbonyl)-amino]-6-methyl-4,9-dioxo-1,5-dioxonan-7-yl 2-methylpropanoate, (4.005) pencycuron, (5.004) chlorothalonil, (5.013) mancozeb, (5.018) propineb, (12.003) metalaxyl, (12.004) metalaxyl-M (mefenoxam), (13.001) fludioxonil, (13.004) proquinazid, (15.008) cyflufenamid, and (15.047) 3-(4,4-difluoro-3,3-dimethyl-3,4-dihydroisoquinolin-1-yl)quinolone.

**10.** Composition for controlling harmful microorganisms in crop protection and in the protection of materials, **characterized by** a content of an active compound combination according to at least one of claims 1 to 9, in addition to at least one extender and/or surfactant.

**11.** Method for controlling harmful microorganisms in crop protection and in the protection of materials, **characterized in that** an active compound combination according to at least one of claims 1 to 9 or a composition according to claim 10 is applied to the harmful microorganisms and/or their habitat.

**12.** Use of an active compound combination according to at least one of claims 1 to 9 or a composition according to claim 10 for control of harmful microorganisms in crop protection and in the protection of materials.

**13.** Use of an active compound combination according to at least one of claims 1 to 9 or a composition according to claim 10 for treatment of a transgenic plant.

**14.** Use of an active compound combination according to at least one of claims 1 to 9 or a composition according to claim 10 for treatment of seed.

**15.** Seed coated with an active compound combination according to at least one of claims 1 to 9 or a composition according to claim 10.

Europäisches Patentamt
European Patent Office
Office européen des brevets

# EUROPEAN SEARCH REPORT

Application Number
EP 18 21 2562

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/204435 A1 (DOW AGROSCIENCES LLC [US]) 8 November 2018 (2018-11-08) * Mefentrifluconazole+Compound I; claims 1, 2, 8, 21-23; tables 6, 7 * ----- | 1-15 | INV. A01N43/653 C07D401/06 C07D249/08 C07D211/74 |
| X | anonymous: "Fungicidal Compositions", IP.com journal, 24 January 2018 (2018-01-24), pages 1-15, XP055553223, ISSN: 1533-0001, DOI: https://priorart.ip.com/IPCOM/000252556 Retrieved from the Internet: URL:https://priorart.ip.com/IPCOM/000252556 [retrieved on 2019-02-07] * page 11 - page 12 * ----- | 1-15 | |
| X | WO 2018/202428 A1 (BASF SE [DE]) 8 November 2018 (2018-11-08) * claim 1 * ----- | 1-15 | |

TECHNICAL FIELDS SEARCHED (IPC)

A01N
C07D

The present search report has been drawn up for all claims

1

| Place of search | Date of completion of the search | Examiner |
|---|---|---|
| Munich | 7 February 2019 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 21 2562

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2019

| Patent document cited in search report | Publication date | Patent family member(s) | Publication date |
|---|---|---|---|
| WO 2018204435 A1 | 08-11-2018 | UY 37716 A | 30-11-2018 |
| | | WO 2018204435 A1 | 08-11-2018 |
| WO 2018202428 A1 | 08-11-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0275955 A
- CN 101225074 A
- DE 4003180 A
- EP 0113640 A
- EP 0470466 A
- US 4949720 A
- EP 0126430 A
- DE 3801233 A
- WO 2013007767 A
- WO 2013010862 A
- WO 2013010885 A
- WO 2013010894 A
- WO 2013024075 A
- WO 2013024076 A
- WO 2013024077 A
- WO 2013024080 A
- WO 2013024081 A
- WO 2013024082 A
- WO 2013024083 A
- WO 2014082872 A
- WO 2010146114 A
- WO 2010146116 A
- WO 2017029179 A
- WO 200364572 A


### Non-patent literature cited in the description


- *J. Agric. Food Chem.,* 2009, vol. 57, 4854-4860
- *J. Med. Chem.,* 1995, vol. 38 (11), 1892-1903
- *J. Heterocyc. Chem.,* 1981, vol. 18 (7), 1305-1308
- *J. Am. Chem. Soc.,* 2001, vol. 123 (25), 5962-5973
- *CHEMICAL ABSTRACTS,* 1961312-55-9
- Pesticide Manual
- **COLBY, S.R.** Calculating Synergistic and Antagonistic Responses of Herbicide Combinations. *Weeds,* 1967, vol. 15, 20-22
- **TAMMES.** Isoboles, a graphic representation of synergism in pesticides. *Neth. J. Plant Path.,* 1964, vol. 70, 73-80